(19) Europäisches Patentamt / European Patent Office / Office européen des brevets

(11) **EP 1 788 942 B1**

(12) **EUROPÄISCHE PATENTSCHRIFT**

(45) Veröffentlichungstag und Bekanntmachung des Hinweises auf die Patenterteilung:
**14.07.2010   Patentblatt 2010/28**

(51) Int Cl.:
*A61B 5/08* $^{(2006.01)}$      *G06F 17/00* $^{(2006.01)}$
*A61B 5/091* $^{(2006.01)}$

(21) Anmeldenummer: **05791321.2**

(22) Anmeldetag: **11.08.2005**

(86) Internationale Anmeldenummer:
**PCT/EP2005/008768**

(87) Internationale Veröffentlichungsnummer:
**WO 2006/018237 (23.02.2006 Gazette 2006/08)**

(54) **VORRICHTUNG UND VERFAHREN ZUR ERFASSUNG UND AUSWERTUNG VON EXPIROGRAMMEN**

DEVICE AND METHOD FOR THE DETECTION AND EVALUATION OF EXPIROGRAMS

DISPOSITIF ET PROCEDE DE DETECTION ET D'EVALUATION D'EXPIROGRAMMES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **12.08.2004   DE 102004039194**

(43) Veröffentlichungstag der Anmeldung:
**30.05.2007   Patentblatt 2007/22**

(73) Patentinhaber: **Universitätsklinikum Freiburg**
**79106 Freiburg (DE)**

(72) Erfinder: **RÖCKER, Kai**
**79102 Freiburg (DE)**

(74) Vertreter: **Müller-Boré & Partner**
**Patentanwälte**
**Grafinger Strasse 2**
**81671 München (DE)**

(56) Entgegenhaltungen:
**US-A- 5 971 934**

- **KARS A H ET AL: "Dead space and slope indices from the expiratory carbon dioxide tension-volume curve." THE EUROPEAN RESPIRATORY JOURNAL : OFFICIAL JOURNAL OF THE EUROPEAN SOCIETY FOR CLINICAL RESPIRATORY PHYSIOLOGY. AUG 1997, Bd. 10, Nr. 8, August 1997 (1997-08), Seiten 1829-1836, XP002362086 ISSN: 0903-1936**
- **NEUFELD G R ET AL: "Modelling steady state pulmonary elimination of He, SF6 and CO2: effect of morphometry." RESPIRATION PHYSIOLOGY. JUN 1992, Bd. 88, Nr. 3, Juni 1992 (1992-06), Seiten 257-275, XP002362087 ISSN: 0034-5687**
- **HUANG J W ET AL: "Airway cross section strongly influences alveolar plateau slope of capnograms for smaller tidal volumes." RESPIRATION PHYSIOLOGY. JAN 2000, Bd. 119, Nr. 1, Januar 2000 (2000-01), Seiten 51-55, XP002362088 ISSN: 0034-5687**
- **VON BASUM GOLO ET AL: "Online recording of ethane traces in human breath via infrared laser spectroscopy." JOURNAL OF APPLIED PHYSIOLOGY (BETHESDA, MD. : 1985) DEC 2003, Bd. 95, Nr. 6, Dezember 2003 (2003-12), Seiten 2583-2590, XP002362089 ISSN: 8750-7587**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zur Erfassung und Auswertung von Expirogrammen, ein Verfahren zur Erfassung und Auswertung von Expirogrammen sowie ein zugeordnetes Computerprogrammprodukt.

Formeigenschaften sogenannter Expirogramme sind insbesondere für wissenschaftliche und diagnostische Zwecke von grundsätzlichem Interesse. Unter einem Expirogramm wird der charakteristische Verlauf der Gaskonzentration der Atemgase Kohlendioxid ($CO_2$) und Sauerstoff ($O_2$) gegen das Atemvolumen bzw. gegen die Zeit verstanden. So wird mit jeder Ausatmung (Expiration) Kohlendioxid aus dem Körper entfernt und Sauerstoff aus der Luft in den Blutkreislauf eingebracht. Im Verlaufe jeder Ausatmung ergibt sich in Abhängigkeit des ausgeatmeten Atemvolumens ein charakteristischer Verlauf der Gaskonzentrationen dieser beiden Atemgase.

Bei Säugetieren und Menschen erfolgt die Luftzufuhr über den Mund oder die Nase durch die Luftröhre. Der Kehlkopf ist in der Lage, die Luftröhre gegen den Schlund, z.B. beim Schlucken, zu verschließen. Die Luftröhre gabelt sich in die Hauptbronchien, die in die Lungen hineinführen. Dort spalten sich die Hauptbronchien in immer dünnere Bronchien auf, die über sehr feine Bronchien, den sogenannten Broncheoli, in den Alveolarkanälen und schließlich in kleinen Bläschen, den Alveolen enden. Die respiratorischen Epithelien werden somit in einer bidirektionellen Wechselströmung (Ein- und Ausatmen) ventiliert.

Besondere Bedeutung kommt in diesem Zusammenhang dem sogenannten "Totraum" einer Lunge zu. Als Totraum wird derjenige Rauminhalt der Lunge bezeichnet, welcher zwar mit der Außenluft in Verbindung steht und von dieser belüftet werden kann, jedoch nicht am Gasaustausch mit den Körpergeweben teilnimmt. Zum Totraum gehören bei Gesunden insbesondere die Transportwege der Atemluft, wie die Luftröhre und die Luftröhrenäste (Bronchien).

Das Gegenstück zum Totraum der Lunge ist derjenige Rauminhalt, aus welchem Gasaustausch zwischen Kapillaren und Außenluft stattfindet. Bei diesem Austausch wird Luftsauerstoff in das Blut aufgenommen und Kohlendioxid in den Lungenraum hinein abgegeben. Dieser Raum, in welchem der Gasaustausch stattfindet, hat die Struktur kleiner Bläschen, welche als sogenannte Alveolen bezeichnet werden. Die Alveolen sind als Ort des Gasaustauschs reich mit kleinsten Blutgefäßen, den Kapillaren, versorgt. Zwischen diesen Kapillaren und dem Luftraum der Alveole liegt eine Gewebsschicht von weniger als 1 $\mu$m Dicke, die sogenannte Blut-Luft-Schranke. Hier erfolgt die Aufnahme von $O_2$ aus der eingeatmeten Luft in das Blut, in dem es durch das Atmungspigment Hämoglobin gebunden wird, und die Abgabe von $CO_2$ aus dem Blut in die Luft entlang dem jeweiligen Partialdruckgefälle durch Diffusion. $CO_2$ liegt im Blut gelöst als $HCO_3^-$ vor.

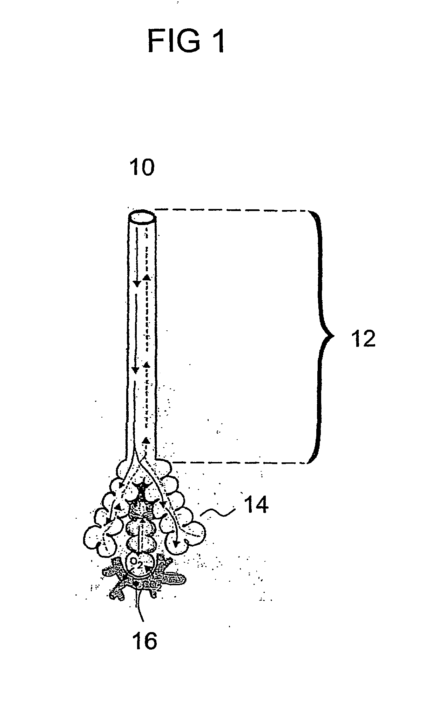

Fig. 1 zeigt in stark schematisierter Form den Weg der Atemluft in die Alveolen. Außenluft 10 gelangt über einen Atemweg 12 in die Alveolen 14, in welchen der Austausch der Atemgase $O_2$ und $CO_2$ mit Kapillaren 16 des Blutsystems erfolgt.

Ein Austausch der Atemgase $O_2$ und $CO_2$ findet immer in den Alveolen statt. Andererseits können auch belüftete Alveolen funktionell zu "Totraum" werden, wenn sie zwar belüftet, jedoch nicht in ausreichendem Maße von Blut umströmt werden. Auch ist der belüftete Anteil der Lunge keine konstante Größe. Mit zunehmender Atemzugtiefe werden Abschnitte mit zusätzlichem Totraum und zusätzlichem Austauschraum belüftet und umgekehrt. Selbst bei maximaler Ausatmung bleibt immer ein Residualvolumen in der Lunge zurück.

Der sogenannte "physiologische Totraum" $x_{VD}$ ist hierbei derjenige Totraum, welcher mit jedem Atemzug ein neues Volumen einnimmt. Demgegenüber ist der sogenannte "anatomische Totraum" die Summe des Volumens aller anatomischen Strukturen der Atemwege, welche nicht am Gasaustausch teilnehmen können - unabhängig davon, ob sie aktuell belüftet werden oder nicht. Die sogenannte Atemzugtiefe $x_{VT}$ ist das gesamte Gasvolumen einer einzelnen Ausatmung (Expiration). Der Austauschraum oder der Alveolarraum ($x_{VA}$) ist somit die Differenz zwischen der Atemzugtiefe $x_{VT}$ und dem physiologischen Totraum $x_{VD}$:

$$x_{VA} = x_{VT} - x_{VD} \tag{1}$$

Den charakteristischen Verlauf der Konzentration der beiden Atemgase $O_2$ und $CO_2$ während der Ausatmung gegen das Volumen und gegen die Zeit wird in einem Expirogramm dargestellt. Mit Hilfe eines solchen Expirogramms läßt sich der Totraum einer Lunge bestimmen. Dies kann in verschiedenen medizinischen Bereichen genutzt werden. So können Expirogramme beispielsweise eine nichtinvasive Methode für das Erkennen von krankhaften Veränderungen der Lunge darstellen. Dies kann für die Beobachtung von Personen mit einem erhöhten Risiko einer krankhaften Veränderung der Lunge, beispielsweise erblich vorbelasteten Personen oder Rauchern, genutzt werden. Zudem können bei einer erkannten krankhaften Veränderung der Lunge therapeutische Erfolge mit Hilfe von regelmäßig durchgeführten Expirogrammen beobachtet werden. Auch eine Verminderung des Atemzugvolumens beispielsweise aufgrund von neuromuskulären Störungen kann mit Hilfe des Expirogramms detektiert werden.

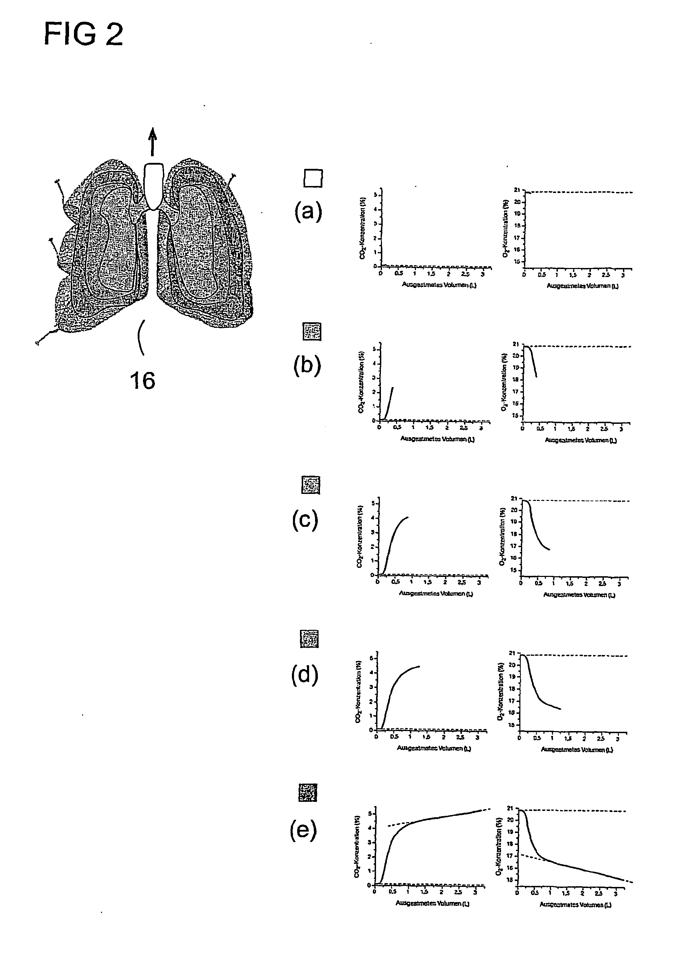

Die Form der Expirogramme zeigen aufgrund der Lungenanatomie unterschiedliche, charakteristische Verläufe, welche beispielhaft in **Fig. 2** dargestellt sind. In Fig. 2 (a) bis (e) sind die am Mund gemessenen Konzentrationen der Atemgase $CO_2$ (linke Spalte) und $O_2$ (rechte Spalte) während der Ausatmung gegen das Atemvolumen der ausgeatmeten Luft aufgetragen. Mit Beginn der Expiration (vgl. Fig. 2(a)) strömt der Gasinhalt von Mund, Rachen und oberer Luftröhre am Mund vorbei. In diesem Atemvolumenbereich findet kein Gasaustausch über die Alveolen statt, so daß die Konzentrationsverteilung der ausgeatmeten Luft der zuvor eingeatmeten Raumluftzusammensetzung entspricht. Mit zunehmendem ausgeatmeten Volumen nimmt jedoch der Anteil der sogenannten Alveolarluft, d.h. der Luft aus dem Austauschraum der Lunge, am Mund fortlaufend zu, womit die $CO_2$-Konzentration steigt und die $O_2$-Konzentration absinkt. Figs. 2(b) bis (d) zeigen den mit der Ausatmung aus zunehmend tieferen Lungenregionen verbundenen Anstieg der $CO_2$-Konzentration und entsprechenden Abfall der $O_2$-Konzentration in der ausgeatmeten Atemluft. Der Graustufeninset zeigt schematisch die Lungenregion der Lunge 18 an, deren Gasinhalt aktuell zur Ausatmung beiträgt.

Je mehr vom Inhalt des Totraums ausgewaschen ist, desto näher rücken die am Mund gemessenen Gaskonzentrationen an diejenigen Gaskonzentrationen im Austauschraum bzw. Alveolarraum. Sobald der Totraum komplett ausgewaschen ist, entsprechen die Gaskonzentrationen am Mund denjenigen Gaskonzentration im Alveolarraum (vgl. Fig. 2(e)). Die alveolare Gaskonzentration wiederum verändert sich im Verlauf der Ausatmung gegenüber dem Atemvolumen in linearer Weise, so daß der Kurvenverlauf des Expirogramms für hohe Atemvolumenwerte in eine Gerade übergeht. Dieser lineare Verlauf des Expirogramms ist für $CO_2$ durch den Umstand begründet, daß sich ein konstant nachströmender $CO_2$-Fluß in ein - der Ausatmung entsprechend - geringer werdendes Alveolenvolumen einmischt. Für $O_2$ gilt der umgekehrte Fall, gemäß welchem aus zunehmend kleinerem Alveolenvolumen Sauerstoff in konstantem Fluß entfernt wird. Diese linearen Anteile der Expirogramme werden aufgrund ihrer Herkunft oftmals mit "Alveolar Slopes" bezeichnet.

Naturgemäß ist die Information des physiologischen Totraums $x_{VD}$ eines Atemzugs in dem Kurvenverlauf jedes Expirogramms enthalten. Je näher die gemessene Gaskonzentration an die alveolare Gaskonzentration heranrückt, desto mehr Totraum ist ausgewaschen bzw. desto weniger Totraum ist noch nicht mit Alveolarluft gefüllt. Ist die alveolare Gaskonzentration im Verlauf bekannt, kann aus der Verteilungsfunktion bis zum Erreichen der alveolaren Gaskonzentration der physiologische Totraum $x_{VD}$ bestimmt werden. Der physiologische Totraum $x_{VD}$ entspricht dann der mittleren Auswaschung des Totraums mit Alveolarluft.

Ein zuverlässiges, robustes und numerisch aussagekräftiges Analyseverfahren zur Ermittlung der sogenannten "Alveolar Slopes" ist bislang nicht bekannt.

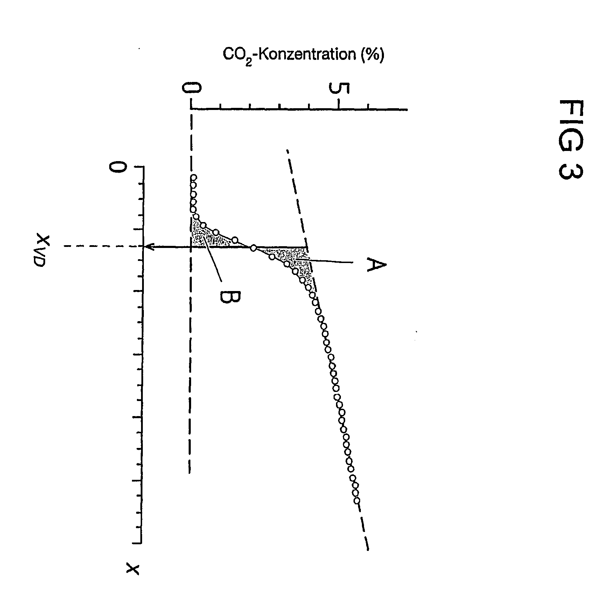

zur Bestimmung des physiologischen Totraums $x_{VD}$ bedient man sich herkömmlicher Weise dem Verfahren nach Fowler et al., welches 1948 in "The Respiratory Dead Space", Am J Physiol 154: Seiten 405 - 416 vorgestellt wurde. Das Bestimmungsverfahren des physiologischen Totraums $x_{VD}$ nach Fowler ist schematisch in **Fig. 3** für $CO_2$ dargestellt. Um den physiologischen Totraum $x_{VD}$ bestimmen zu können, wird der linear ansteigende Kurventeil des Expirogramms mit einer Geraden gefittet. Über einen Flächenvergleich des nichtlinearen Kurvenanteils des Expirogramms unter Zuhilfenahme der zuvor bestimmten Fitgerade des linearen Kurvenanteils erfolgt die Bestimmung des physiologischen Totraums $x_{VD}$ (gemessen in Litern Atemvolumen). Der physiologische Totraum $x_{VD}$ ist dasjenige ausgeatmete Atemvolumen, bei welchem die in Fig. 3 dargestellten Flächen A und B den gleichen Flächeninhalt aufweisen. Aufgrund des oftmals problematischen Fittings der Geraden in den linearen Kurvenanteil von Expirogrammen ist das herkömmliche Fowler-Verfahren jedoch in besonderer Weise numerisch empfindlich, so daß eine zuverlässige Auswertung des physiologischen Totraums $x_{VD}$ aus Expirogrammen nur bedingt möglich ist.

Andere Methoden zur in-vivo Bestimmung des physiologischen Totraums werten beispielsweise den $CO_2$ freien Anteil des ausgeatmeten Volumens aus ("Bohr dead space") oder den Mittelwert der Verteilungsfunktion des $CO_2$ Partialdrucks in Abhängigkeit vom Exspirationsvolumen (PIE). Ein Vergleich dieser Methoden findet sich in "Dead space and slope indices from the expiratory carbon dioxide tension-volume curve" (A.H. Kars et. al., Eur Respir J 1997; 10: 1829-1836).

Aufgabe der Erfindung ist es, eine Vorrichtung zur Erfassung und Auswertung von Expirogrammen anzugeben, welche in numerisch zuverlässiger, reproduzierbarer und robuster Weise Expirogramme erfassen und auswerten kann, um den charakteristischen Kurvenverlauf des Expirogramms bestimmen und auswerten zu können. Eine weitere Aufgabe der Erfindung ist die Angabe eines entsprechenden Verfahrens sowie eines entsprechenden Computerprogrammprodukts.

Diese Aufgaben werden durch eine Vorrichtung mit den in Anspruch 1 angegebenen Merkmalen, ein Verfahren mit den in Anspruch 9 angegebenen Verfahrensschritten sowie ein Computerprogrammprodukt mit den im Anspruch 15 angegebenen

Merkmalen gelöst. Bevorzugte Ausführungsformen sind Gegenstand der abhängigen Ansprüche.

Gemäß der Erfindung umfaßt eine Vorrichtung zur Erfassung und Auswertung von Expirogrammen:

- eine Gasmeßsonde, welche zur Ermittlung einer Gaskonzentration $f_{mess}$ eines Gases in ausgeatmeter Atemluft ausgelegt ist;
- eine Ausleseeinheit, welche mit der Gasmeßsonde in Signalverbindung steht und ausgelegt ist, für eine Vielzahl von Werten $x_1,...x_N$ eines ausgeatmeten Volumens der ausgeatmeten Atemluft die zugeordneten ermittelten Gaskonzentrationen $f_{mess}(x_1),..., f_{mess}(x_N)$ aus der Gasmeßsonde auszulesen;

- eine Speichereinheit, welche zur Speicherung der den Werten $x_1,...,x_N$ zugeordneten Gaskonzentrationen $f_{mess}(x_1),...,f_{mess}(x_N)$ ausgelegt ist;

- eine Funktionsfiteinheit, welche mit der Speichereinheit in Signalverbindung steht und ausgelegt ist, eine nichtlineare Fitfunktion

$$f(x) = g(x) \cdot h(x) + \textit{Offset}_{Gas} \qquad (2)$$

für die gespeicherten Gaskonzentrationen $f_{mess}(x_1),...,f_{mess}(x_N)$ durch Bestimmen von Funktionen $g(x)$ und $h(x)$ zu bestimmen, wobei

-- $h(x) = a + b \cdot x$ ist,

-- $g(x)$ eine stetig differenzierbare, nichtlineare Funktion mit g(0) = 0 und $\lim\limits_{x \to \infty} g(x) = const$ ist,

-- $a,b$ und *const* Konstanten sind und

-- *Offset*$_{Gas}$ eine konstante, mittlere Konzentration des Gases in Raumluft ist.

Vorzugsweise umfasst die Vorrichtung eine Funktionsfiteinheit, welche mit der Speichereinheit in Signalverbindung steht und ausgelegt ist, eine nichtlineare Fitfunktion

$$f(x) = g(x) \cdot h(x) + \textit{Offset}_{Gas}$$

für die gespeicherten Gaskonzentrationen $f_{mess}(x_1),...,f_{mess}(x_N)$ zu bestimmen, wobei

-- $h(x) = a+b \cdot x$ ist,

-- $g(x)$ eine stetig differenzierbare, nichtlineare Funktion mit

$g(0) = 0$ und $g(x) = const$ für $x \geq x_{max}$ ist, falls das Gas Kohlendioxid ist bzw.

$g(0) = const$ und $g(x) = 0$ für $x \geq x_{max}$ ist, falls das Gas Sauerstoff ist,

-- a, b, const und $x_{max}$ Konstanten sind und

-- *Offset*$_{Gas}$ eine konstante, mittlere Konzentration des Gases in Raumluft ist.

Mittels der Ausleseeinheit und der damit in Signalverbindung stehenden Meßsonde werden für eine Vielzahl von Volumenwerten $x_1,...,x_N$ eines ausgeatmeten Volumens der ausgeatmeten Atemluft in einem Atemzug die zugeordneten Gaskonzentrationen $f_{mess}(x_1),...,f_{mess}(x_N)$ bestimmt. Die Gasmeßsonde ist ausgelegt, unmittelbar am Mund mit ausreichender Wiederholrate die Gaskonzentration $f_{mess}$ eines Atemgases, d.h. Sauerstoff $O_2$ oder Kohlendioxid $CO_2$ während eines Atemzugs zu messen. Die Wiederholrate beträgt vorzugsweise zumindest 15 Hz, weiter bevorzugt zumindest 25 Hz. Vorzugsweise werden zumindest 15, weiter bevorzugt zumindest 25 Werte *N* erfaßt. Die gemessenen Gaskonzentrationen $f_{mess}(x_1),...,f_{mess}(x_N)$ sowie die zugeordneten Volumenwerte $x_1,...,x_N$ werden in der Speichereinheit abgelegt. Die Volumenwerte werden vorzugsweise für jeden Atemzug anhand eines gemessenen Gasflusses *Flow(t)*, d.h. des Volumens der ausgeatmeten Atemluft pro Zeiteinheit, berechnet, wobei der Volumenwert $x(t_i)$ zum Zeitpunkt $t_i$

das Zeitintegral über den gemessenen Gasfluß *Flow(t)* ist, d.h. $x_i = x(t_i) = \int\limits_0^{t_i} Flow(t)dt$ gilt, wenn die Ausatmung zum dem Zeitpunkt t = 0 erfolgt. Die Messung des Gasflusses *Flow(t)* erfolgt vorzugsweise mittels einer

Gasflußmeßsonde, welcher ausgelegt ist, den Gasfluß in unmittelbarer Nähe des Mundes zu messen. Die Funktionsfiteinheit greift auf die in der Speichereinheit abgelegten Gaskonzentrationen $f_{mess}(x_1),..., f_{mess}(x_N)$ und Volumenwerte $x_1,...,x_N$ zu, um eine nichtlineare Fitfunktion f(x) zu bestimmen.

Gemäß der Erfindung erfolgt das Fitting an die Meßdaten an die in der Speichereinheit gespeicherten Gaskonzentrationen durch eine nichtlineare Fitfunktion *f(x)*, welche als Produktansatz zweier Funktionen mit einem Offset gebildet ist. Beide Produktterme der Fitfunktion *f(x)* stellen physiologisch begründete Anteile der Expirogramme dar und ermöglichen eine überraschend robuste, zuverlässige und numerisch genaue Auswertung des erfaßten Expirogramms.

Im folgenden soll die Funktionsweise der Funktionsfiteinheit sowie die nichtlineare Fitfunktion $f(x)$ anhand des Atemgases $CO_2$ beispielhaft erläutert werden. Sämtliche Ausführungsformen gelten in entsprechender Weise für das Atemgas $O_2$.

Gemäß der Erfindung enthält die nichtlineare Fitfunktion $f(x)$ einen ersten Produktterm $h(x)$, welcher den linear ansteigenden Kurvenanteil des Expirogramms für das Atemgas $CO_2$ beschreibt. Der Produktterm $h(x)$ hat somit die Funktionsgleichung einer Geraden, so daß

$$h(x) = a + b \cdot x \qquad\qquad (3)$$

gilt.

Der zweite Produktterm $g(x)$ der nichtlinearen Fitfunktion $f(x)$ stellt den anteiligen Verlauf der Totraumauswaschung bis zum Erreichen der 100%-igen Alveofarluftkonzentration am Mund dar. Bei der Funktion $g(x)$ handelt es sich um eine stetig differenzierbare Funktion, d.h. eine Funktion, deren erste Ableitung nach dem Atemvolumen eine stetige Funktion ergibt, welche zusätzlich folgenden Bedingungen genügen muß:

Falls das Atemgas Kohlendioxid ist, ist der Funktionswert für das Atemvolumen 0 (Beginn der Expiration) gleich 0. Für große x nähert sich die Funktion $g(x)$ einem konstanten Wert. Oberhalb eines gewissen Atemvolumens $x_{max}$ ist die Funktion $g(x)$ vorzugsweise eine konstante Funktion, wobei vorzugsweise $g(x) = 1$ für $x \geq x_{max}$ gilt.

Zwischen dem Volumenwert $x = 0$, bei welchem keine Luft aus dem Alveolarraum den Mund erreicht, bis zu dem Volumenwert $x_{max}$, bei welchem am Mund zu 100% die Alveolarluftkonzentration gemessen wird, steigt die Funktion $g(x)$ vorzugsweise monoton an.

Für das Atemgas $O_2$ ergibt sich ein entsprechend umgekehrter Verlauf für die Funktion $f(x)$. Dieser umgekehrte Verlauf wird durch ein entgegengesetztes Vorzeichen der Konstante $b$ aus der linearen Funktion $h(x)$ bewirkt. Insbesondere ist der Funktionswert der Funktion $f(x)$ für das Atemvolumen $x = 0$ ein vorbestimmter konstanter Wert.

Die Parameter $a$, $b$ des linearen Produktterms $h(x)$ sowie das Grenzvolumen $x_{max}$ stellen hierbei Konstanten dar, welche beim Fitting zu bestimmen sind. Ferner weist die nicht lineare Fitfunktion $f(x)$ einen konstanten Offset auf, welcher die (konstante) mittlere Konzentration des jeweiligen Atemgases in der Raumluft darstellt. Das Vorzeichen der Steigung $b$ des linearen Produkttherms $h(x)$ der nichtlinearen Fitfunktion $f(x)$ ist für das Atemgas $CO_2$ positiv, während es für das Atemgas $O_2$ negativ ist.

Die Funktionsfiteinheit ist ausgelegt, unter Zuhilfenahme eines bekannten Fitalgorithmus die gespeicherten, gemessenen Expirogrammdaten $(x_1,..-,x_N; f_{mess}(x_1),..., f_{mess}(x_N))$ mittels der so definierten, nichtlinearen Fitfunktion $f(x)$ anzunähern. Die gemessenen Gaskonzentrationswerte $f_{mess}(x_i)$ werden somit nach erfolgtem Fitting im wesentlichen den Werten $f(x_i)$ entsprechen, wobei $1 \leq i \leq N$ gilt. Durch das nichtlineare Fitting der Expirogrammdaten mittels eines Produktansatzes mit den physiologisch begründeten Produkttermen $g(x)$ und $h(x)$ wird eine robuste, einfache und reproduzierbare Bewertung von Formeigenschaften des erfaßten Expirogramms möglich.

Gemäß einer besonders bevorzugten Ausführungsform umfaßt die erfindungsgemäße Vorrichtung ferner eine Auswerteeinheit, welche zur Bestimmung des physiologischen Totraums $x_{VD}$ einer zugeordneten Lunge ausgelegt ist, wobei

$$\int_0^{x_{VD}} g(x)h(x)\,dx = \lim_{x_{max}\to\infty} \int_{x_{VD}}^{x_{max}} h(x)(1-g(x))\,dx \qquad\qquad (4)$$

gilt. Für das Atemgas Kohlendioxid beträgt der Funktionswert $f(0) = Offset_{co2}$.

Alternativ umfaßt eine bevorzugte Ausführungsform der erfindungsgemäßen Vorrichtung eine Auswerteeinheit, welche zur Bestimmung des physiologischen Totraums $x_{VD}$ einer zugeordneten Lunge ausgelegt ist, wobei

$$\int_0^{x_{VD}} g(x)h(x)\,dx - f(0)x_{VD} = \int_{x_{VD}}^{x_{max}} h(x)(1-g(x))\,dx$$

gilt.

Aufgrund des stabilen Funktionsfittings mittels der aus einem Produktansatz gebildeten, nicht linearen Fitfunktion läßt sich in einfacher und numerisch stabiler Weise der physiologische Totraum $x_{VD}$ einer zugeordneten Lunge, aus welcher die Atemluft ausgeatmet wurde, bestimmen. Die Bestimmung erfolgt mittels einer Auswerteeinheit, welche zur Lösung der Integralgleichung (4) nach dem physiologischen Totraum $x_{VD}$ ausgelegt ist. Hierbei können sämtliche herkömmlichen Verfahren zur numerischen Lösung von Integralgleichungen herangezogen werden, wie sie in der numerischen Mathematik wohlbekannt sind.

Im Vergleich zu dem herkömmlichen Bestimmungsverfahren des physiologischen Totraums $x_{VD}$ nach Fowler et al. ermöglicht die bevorzugte erfindungsgemäße Vorrichtung eine erheblich genauere, reproduzierbarere und weniger von der Meßqualität der Expirogrammdaten abhängige Bestimmung des physiologischen Totraums $x_{VD}$. Hierfür ist in erster Linie der gewährte erfindungsgemäße Produktansatz der Fitfunktion $f(x)$ verantwortlich, welcher aus den eingangs genannten Produkttermen gebildet wird. Die bevorzugte erfindungsgemäße Vorrichtung gestattet es, eine Vielzahl von Expirogrammen automatisch auszuwerten und den jeweiligen physiologischen Totraum $x_{VD}$ für jeden Atemzug, d.h. für jedes Expirogramm, zu bestimmen.

Vorzugsweise ist die Funktion g(x) eine monotone Funktion. Besonders bevorzugt gilt

$$g(x) = \frac{k_4}{1 + \dfrac{e^{(k_1 \cdot c - k_1 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} - \dfrac{e^{(k_2 \cdot c - k_2 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} + k_3 \qquad (5)$$

und am meisten bevorzugt

$$g(x) = \frac{1}{1 + \dfrac{e^{(k_1 \cdot c - k_1 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} - \dfrac{e^{(k_2 \cdot c - k_2 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} + k_3$$

wobei $k_1 > k_2, k_3, k_4$ und $c$ Konstanten sind. $k_3$ *und* $k_4$ werden dabei vorzugsweise so gewählt, daß $g(0) = 0$ und $\lim_{x \to \infty} g(x) = 1$ gilt.

$k_1$ stellt hierbei die Steilheit des unteren Kurvenanteils, $k_2$ die Steilheit des oberen Kurvenanteils und $c$ die Lage der Kurve in der x-Richtung dar. Der Term $k_3$ vorzugsweise derart gewählt, daß $g(0) = 0$ gilt. x ist wiederum das aktuelle, innerhalb eines Atemzuges ausgeatmete Atemvolumen, wobei auch eine Auftragung gegen die Zeit der Ausatmung möglich ist.

Alternativ gilt vorzugsweise

$$g(x) = \frac{1}{1 + \dfrac{e^{(k_1 \cdot c - k_1 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} - \dfrac{e^{(k_2 \cdot c - k_2 \cdot x)}}{1 + e^{\left(\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}}$$

wobei $k_1, k_2$ und c Konstanten sind.

Die durchschnittliche Steilheit der kurvigen Anteile des Expirogramms ergeben sich nach erfolgtem nichtlinearen Fitting aus

$$Steilheit = \frac{2 \cdot k_1 \cdot k_2}{|k_1 + k_2|} \qquad\qquad (6)$$

Vorzugsweise ist die Funktionsfiteinheit ausgelegt, die Fitfunktion $f(x) = g(x) \cdot h(x) + Offset_{Gas}$ nach dem Marquardt-Levenberg-Algorithmus zu bestimmen. Es hat sich gezeigt, daß nach dem Marquardt-Levenberg-Algorithmus eine besonders gutes nichtlineares Fitting der Fitfunktion an die erfaßten Expirogrammdaten möglich ist.

Vorzugsweise ist die Speichereinheit eine elektronische Speichereinheit. Insbesondere kann es sich bei der elektronischen Speichereinheit um einen flüchtigen oder nichtflüchtigen Halbleiterspeicher oder beispielsweise um ein magnetisches Speichermedium, wie eine Computerfestplatte, handeln.

Gemäß einer weiteren bevorzugten Ausführungsform umfaßt die erfindungsgemäße Vorrichtung ferner eine Auswerteeinrichtung, welche zur Berechnung der Alveolenrekrutierungsrate $g'(x)$ durch Ableiten der Funktion $g(x)$ nach dem ausgeatmeten Volumen x ausgelegt ist.

Die Alveolenrekrutierungsrate $g'(x)$ bezeichnet die Rate des jeweils beim Atemvolumen x zu Entleerung hinzukommenden Alveolarraums. Je höher der Wert für $g'(x)$ ist, desto mehr Gas aus dem Alveolarraum hat bei diesem ausgeatmetem Volumen jeweils den dazugehörenden Totraum ausgewaschen. Eine derartige Auswertung und Bestimmung der Alveolenrekrutierungsrate $g'(x)$ ist von wesentlicher Bedeutung, insbesondere für wissenschaftliche und auch nichtdiagnostische Zwecke.

Die graphische Darstellung und numerische Skalierung der Alveolenrekrutierungsrate $g'(x)$ bedeutet einen wesentlichen Informationsgewinn. Insbesondere können verschiedene pathologische und physiologische Veränderungen der Lunge anhand dieser Form der Darstellung diagnostizierbar sein. Veränderungen in der Atemmechanik oder der Lungenanatomie hätten einen direkten Einfluß auf die Alveolenrekrutierungsrate. Vorzugsweise handelt es sich bei der Anzeigevorrichtung um einen Monitor, auf welchem die Alveolenrekrutierungsrate graphisch, vorzugsweise mit weiteren Parametern des ausgewerteten Expirogramms zeitgleich oder zeitnah zu der Meßwerterfassung ausgegeben werden. Zeitnah bedeutet in diesem Zusammenhang vorzugsweise innerhalb von fünf Sekunden nach abgeschlossener Meßwerterfassung, besonders bevorzugt innerhalb von einer Sekunde nach abgeschlossener Meßwerterfassung.

Gemäß einer weiteren besonders bevorzugten Ausführungsform umfaßt die erfindungsgemäße Vorrichtung ferner eine Anzeigevorrichtung, welche ausgelegt ist, die Alveolenrekrutierung $g'(x)$ graphisch darzustellen.

Gemäß einem weiteren Aspekt der Erfindung umfaßt eine Vorrichtung insbesondere zur Erfassung und Auswertung von Expirogrammen:

- eine Gasflußmeßsonde, welche zur Ermittlung eines Gasflusses $Flow(t)$ (d.h. eines ein- bzw. ausgeatmeten Atemluftvolumens pro Zeiteinheit) von Atemluft in Abhängigkeit der Atemzeit $t$ ausgelegt ist;
- eine Gasmeßsonde, welche zur Ermittlung einer Gaskonzentration $f_{mess}(t)$ eines Gases in der ein- bzw. ausgeatmeten Atemluft ausgelegt ist;
- eine Atemzugerkennungseinheit, welche zur Erkennung eines Beginns $x_B$ einer Einatmung anhand des ermittelten Gasflusses $Flow(t)$ ausgelegt ist;
- eine Extremaerkennungseinheit, welche zur Erkennung von Werten $t_{Extrema}$, für welche $f_{mess}(t_{Extrema})$ Extrema darstellen, anhand der ermittelten Gaskonzentration $f_{mess}(t)$ ausgelegt ist;
- eine Synchronisationseinheit, welche den erkannten Beginn $t_B$ der Einatmung jedes Atemzugs mit dem zugeordneten, erkannten Wert $t_{Extrema} = t_B + \Delta t$ der ermittelten Gaskonzentration durch Verschieben des ermittelten Gasflusses $Flow(t)$ oder Verschieben der ermittelten Gaskonzentration $f_{mess}(t)$ um einen Betrag $\Delta t$ synchronisiert.

Die Ermittlung des Gasflusses $Flow(t)$ und der Gaskonzentration $f_{mess}(t)$ kann hierbei anhand einer Vielzahl diskreter Werte $t_1,...,t_N$ erfolgen, so daß keine kontinuierliche Erfassung notwendig ist. Die Werte $t_1,...,t_N$ stellen Atemzeiten dar, wobei beispielsweise der Zeitpunkt $t = 0$ den Beginn einer Ausatmung eines Atemzugs und $t = t_B$ den Beginn der nachfolgenden Einatmung bezeichnet. Gleichermaßen können obige Zusammenhänge jedoch auch in Abhängigkeit von den Volumina der ausgeatmeten Atemluft dargestellt werden. Die Volumenwerte $x_i$ werden vorzugsweise für jeden Atemzug anhand eines gemessenen Gasflusses $Flow(t)$, d.h. des Volumens der ausgeatmeten Atemluft pro Zeiteinheit, berechnet, wobei der Volumenwert $x(t_i)$ zum Zeitpunkt $t_i$ das Zeitintegral über den gemessenen Gasfluß $Flow(t)$ ist,

$$x_i = x(t_i) = \int_0^{t_i} Flow(t)\,dt \quad \text{gilt, wenn die Ausatmung zum dem Zeitpunkt } t = 0 \text{ erfolgt.}$$

Die Gasflußmeßsonde ist vorzugsweise unmittelbar am Mund des Probanden angeordnet und erfaßt den Gasfluß *Flow(t),* d.h. das ein- bzw. ausgeatmete Atemvolumen pro Zeiteinheit (Einheit Liter/s), als Funktion der Atemzeit *t* bzw. des Atemvolumens. Die Atemzugerkennungseinheit ist zur Erkennung des Beginns $t_B$ der Einatmung anhand des ermittelten Gasflusses *Flow(t)* ausgelegt, wobei vorzugsweise *Flow($t_B$) = 0* und $\left.\dfrac{\partial Flow(t)}{\partial t}\right|_{t_B} > 0$ (Nullstelle der aufsteigenden Flanke von *Flow(t)*). Die Extremaerkennungseinheit ist ausgelegt, diejenigen Werte $t_{Extrema}$ zu erkennen bzw. ermittlen, für welche $f_{mess}(t_{Extrema})$ Extrema aufweisen. Für Kohlendioxid stellen die Extrema Maxima dar (höchste Kohlendioxidkonzentration am Ende der Ausatmung), während für Sauerstoff die Extrema Minima sind (niedrigste Sauerstoffkonzentration am Ende der Ausatmung). Die Extrema fallen mit dem steilen Abfall der Kohlendioxidkonzentration bzw. mit dem steilen Anstieg der Sauerstoffkonzentration von $f_{mess}(t)$ zusammen.

Vorzugsweise findet eine derartige Synchronisierung des ermittelten Gasflusses *Flow(t)* mit der ermittelten Gaskonzentration $f_{mess}(t)$ für jeden Atemzug statt. So hat es sich gezeigt, daß überraschenderweise die Verzögerung $\Delta t$, welche auch als Delay bezeichnet wird, aufgrund von verschiedenen Variablen, insbesondere Luftdruck, Atemaktion, Luftfeuchtigkeit, zeitlich nicht stabil ist, sondern ausgeprägten Schwankungen unterliegen kann. Die physikalische Ursache für die Verzögerung $\Delta t$ liegt insbesondere in dem Umstand begründet, daß der Gasfluß *Flow(t)* und die Gaskonzentration $f_{mess}(t)$ oftmals nicht an dem gleichen Ort meßbar sind. Beispielsweise läßt sich der Gasfluß *Flow(t)* in unmittelbarer räumlicher Nähe zum Mund messen während für die Gaskonzentrationsmessung eine größere, räumlich beabstandet angeordnete Meßsonde nötig ist, welche zusätzliche Schlauch- bzw. Rohrleitungswege für die Atemluft bedingt.

Dieser Aspekt der Erfindung läßt sich mit Vorteil in Zusammenhang mit den zuvorgeannten erfindungsgemäßen Vorrichtungen zur Erfassung und Auswertung von Expirogrammen kombinieren.

Gemäß der Erfindung umfaßt ein Verfahren zur Erfassung und Auswertung von Expirogrammen folgende Schritte:

- Messen von Gaskonzentrationen $f_{mess}(x_1),...,f_{mess}(x_N)$ für eine Vielzahl von Werten $x_1,...,x_N$ eines ausgeatmeten Volumens ausgeatmeter Atemluft;
- Speichern der den Werten $x_1,...,x_N$ zugeordneten, gemessenen Gaskonzentrationen $f_{mess}(x_1),...,f_{mess}(x_N)$;
- Bestimmen einer nichtlinearen Fitfunktion

$$f(x) = g(x) \cdot h(x) + Offset_{Gas}$$

.

für die gespeicherten Gaskonzentrationen $f_{mess}(x_1),...,f_{mess}(x_N)$ durch Bestimmen von Funktionen $g(x)$ und $h(x)$, wobei

-- h(x)=a+b·x ist,

-- $g(x)$ eine stetig differenzierbare, nichtlineare Funktion mit $g(0) = 0$ und $\lim\limits_{x \to \infty} g(x) = const$ ist,

-- *a,b* und *const* Konstanten sind und
-- $Offset_{Gas}$ eine konstante, mittlere Konzentration des Gases in Raumluft ist.

Vorzugsweise umfaßt das Verfahren das Bestimmen einer nichtlinearen Fitfunktion

$$f(x) = g(x) \cdot h(x) + Offset_{Gas}$$

.

für die gespeicherten Gaskonzentrationen $f_{mess}(x_1),...,f_{mess}(x_N)$, wobei

-- *h(x)=a+b·x* ist,
-- *g(x)* eine stetig differenzierbare, nichtlineare Funktion mit
$g(0) = 0$ und *g(x) = const* für $x \geq x_{max}$ ist, falls das Gas Kohlendioxid ist bzw.

$g(0)$ = *const* und g(x) = 0 für $x \geq x_{max}$ ist, falls das Gas Sauerstoff ist,
-- *a, b, const* und $x_{max}$ Konstanten sind und
-- $Offset_{Gas}$ eine konstante, mittlere Konzentration des Gases in Raumluft ist.

Hinsichtlich der Merkmale, Eigenschaften und Vorteile des erfindungsgemäßen Verfahrens wird auf die Beschreibung der erfindungsgemäßen Vorrichtung und deren bevorzugte Ausführungsformen verwiesen. Merkmale, welche lediglich im Zusammenhang mit der erfindungsgemäßen Vorrichtung beschrieben wurden, können auch für das erfindungsgemäße Verfahren Verwendung finden.

Die Durchführung der erfindungsgemäßen Verfahren erfordert von dem Benutzer des Verfahrens keine medizinischen Fachkenntnisse, so daß auch medizinisch nicht- oder gering geschultes Personal mit der Durchführung des Verfahrens betraut werden kann. Die gewonnen Daten können für wissenschaftliche, nichtdiagnostische Zwecke, sowie für einer spätere Diagnosestellung durch einen Arzt verwendet werden.

Vorzugsweise umfaßt das erfindungsgemäße Verfahren weiter den Schritt des Bestimmens des physiologischen Totraums $x_{VD}$ einer zugeordneten Lunge, wobei

$$\int_0^{x_{VD}} g(x)h(x)dx = \lim_{x_{max} \to \infty} \int_{x_{VD}}^{x_{max}} h(x)(1 - g(x))dx \qquad (7)$$

gilt.

Alternativ umfaßt das erfindungsgemäße Verfahren vorzugsweise den Schritt des Bestimmens des physiologischen Totraums $x_{VD}$ einer zugeordneten Lunge, wobei

$$\int_0^{x_{VD}} g(x)h(x)dx - f(0)x_{VD} = \int_{x_{VD}}^{x_{max}} h(x)(1 - g(x))dx$$

gilt.

Vorzugsweise gilt

$$g(x) = \cfrac{1}{1 + \cfrac{e^{(k_1 \cdot c - k_1 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} - \cfrac{e^{(k_2 \cdot c - k_2 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} + k_3 \, ,$$

$$(8)$$

wobei $k_1, k_2, k_3$ und $c$ Konstanten sind.

Alternativ gilt vorzugsweise

$$g(x) = \cfrac{1}{1 + \cfrac{e^{(k_1 \cdot c - k_1 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} - \cfrac{e^{(k_2 \cdot c - k_2 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} \, , \qquad (8)$$

wobei $k_1, k_2$ und $c$ Konstanten sind.

Gemäß einer bevorzugten Variante des erfindungsgemäßen Verfahrens umfaßt der Schritt des Bestimmens der Fitfunktion $f(x)=g(x) \cdot h(x)+Offset_{Gas}$ einen Marquardt-Levenberg-Algorithmus.

Besonders bevorzugt umfaßt das erfindungsgemäße Verfahren ferner den Schritt des Berechnens der Alveolenrekrutierungsrate $g'(x)$ durch Ableiten der Funktion $g(x)$. Vorzugsweise erfolgt ein graphisches Darstellen der Alveolenrekrutierungsrate $g'(x)$ auf einer Anzeigevorrichtung.

Gemäß der Erfindung umfaßt ein Computerprogrammprodukt bzw. Computerprogrammerzeugnis zur Auswertung von Expirogrammen Programmteile zur Durchführung eines Verfahrens mit folgenden Schritten:

- Eingeben von Gaskonzentrationen $f_{mess}(x_1),..., f_{mess}(x_N)$ für eine Vielzahl von Werten $x_1,...,x_N$ eines ausgeatmeten Volumens ausgeatmeter Atemluft;
- Speichern der den Werten $x_1,...,x_N$ zugeordneten, gemessenen Gaskonzentrationen $f_{mess}(x_1),..., f_{mess}(x_N)$;
- Bestimmen einer nichtlinearen Fitfunktion

$$f(x) = g(x) \cdot h(x) + Offset_{Gas}$$

für die gespeicherten Gaskonzentrationen $f_{mess}(x_1),..., f_{mess}(x_N)$ durch Bestimmen von Funktionen $g(x)$ und $h(x)$, wobei

-- $h(x)=a+b \cdot x$ ist,

-- $g(x)$ eine stetig differenzierbare, nichtlineare Funktion mit $g(0) = 0$ und $\lim\limits_{x \to \infty} g(x) = const$ ist,

-- $a,b$ und $const$ Konstanten sind und

-- $Offset_{Gas}$ eine konstante, mittlere Konzentration des Gases in Raumluft ist.

Vorzugsweise umfaßt das Computerprogrammprodukt Programmteile zum Bestimmen einer nichtlinearen Fitfunktion

$$f(x) = g(x) \cdot h(x) + Offset_{Gas}.$$

für die gespeicherten Gaskonzentrationen $f_{mess}(x_1),...,f_{mess}(x_N)$, wobei

-- $h(x)=a+b \cdot x$ ist,

-- $g(x)$ eine stetig differenzierbare, nichtlineare Funktion mit
$g(0) = 0$ und $g(x) = const$ für $x \geq x_{max}$ ist, falls das Gas Kohlendioxid ist bzw.
$g(0) = const$ und $g(x) = 0$ für $x \geq x_{max}$ ist, falls das Gas Sauerstoff ist,

-- $a,b,const$ und $x_{max}$ Konstanten sind und

-- $Offset_{Gas}$ eine konstante, mittlere Konzentration des Gases in Raumluft ist.

Vorzugsweise umfaßt das erfindungsgemäße Computerprogrammprodukt Programmteile zur Durchführung eines Verfahrens mit dem Schritt des Bestimmens des physiologischen Totraums $x_{VD}$ einer zugeordneten Lunge, wobei

$$\int_0^{x_{VD}} g(x)h(x)dx = \lim_{x_{max} \to \infty} \int_{x_{VD}}^{x_{max}} h(x)(1-g(x))dx$$

gilt.

Alternativ umfaßt das erfindungsgemäße Computerprogrammprodukt vorzugsweise Programmteile zur Durchführung eines Verfahrens mit dem Schritt des Bestimmens des physiologischen Totraums $x_{VD}$ einer zugeordneten Lunge, wobei

$$\int_0^{x_{VD}} g(x)h(x)dx - f(0)x_{VD} = \int_{x_{VD}}^{x_{max}} h(x)(1 - g(x))dx$$

gilt.

Hinsichtlich der Merkmale, Vorteile und Eigenschaften des erfindungsgemäßen Computerprogrammprodukts wird auf die zuvorige Beschreibung des erfindungsgemäßen Verfahrens sowie auf die Beschreibung der erfindungsgemäßen Vorrichtung und deren bevorzugter Ausführungsvarianten verwiesen. Merkmale, welche lediglich in Zusammenhang mit dem erfindungsgemäßen Verfahren oder der erfindungsgemäßen Vorrichtung beschrieben werden, können auch für das erfindungsgemäße Computerprogrammprodukt Verwendung finden.

Sowohl für die erfindungsgemäßen Verfahren als auch für die erfindungsgemäßen Computerprogrammprodukte kann mit Vorteil das erfindungsgemäße Konzept einer Synchronisierung des Gasflusses *Flow*(x) mit der Gaskonzentration $f_{mess}(x)$ eingesetzt werden. Insbesondere erfolgt die Berechnung der Verzögerung $\Delta x$ vorzugsweise für jeden Atemzug.

Die Erfindung wird nachfolgend beispielhaft anhand begleitender Zeichnungen beschrieben. Es zeigt:

Fig. 1      eine stark schematische Darstellung des Wegs von Atemluft in die Alveolen;

Fig. 2      die am Mund während der Ausatmung gemessene Konzentration der Atemgase $O_2$ und $CO_2$ gegen das Volumen der ausgeatmeten Luft, wobei die linke Graphenspalte den Verlauf für das Atemgas $CO_2$ und die rechte Graphenspalte den Verlauf für das Atemgas $O_2$ darstellt;

Fig. 3      einen beispielhaften, gemessenen Verlauf der $CO_2$-Konzentration gegen das ausgeatmete Volumen von Atemluft sowie eine Fitgerade zur Bestimmung des physiologischen Totraums $x_{VD}$ gemäß Fowler et al.;

Fig. 4      einen beispielhaften experimentellen Verlauf der $CO_2$-Konzentration gegen das ausgeatmete Atemvolumen, wobei

Fig. 4(a)      den linearen Produktterm h(x) der nichtlinearen Fitfunktion f(x) nach erfolgtem Fitting der Expirogrammdaten darstellt,

Fig. 4(b)      den nichtlinearen Produktterm g(x) der nichtlinearen Fitfunktion f(x) nach erfolgtem Fitting der Expirogrammdaten darstellt;

Fig.4(c)      die nichtlineare Fitfunktion f(x) das offset-korrigierte Produkt der Funktionen h(x) und g(x) darstellt;

Fig. 5      sechs zufällig ausgewählte Einzelbeispiele von Expirogrammen gemeinsam mit den Produktthermen h(x) und g(x) der Fitfunktion f(x)

Fig. 6      nach erfolgtem Fitting, wobei die Fitparameter neben den jeweiligen Expirogramme angegeben sind; die anteilige Alveolenrekrutierung g(x) sowie die Alveolenrekrutierungsrate g'(x) aufgetragen gegen das ausgeatmete Volumen innerhalb eines Atemzugs für die expiratorische $CO_2$- Konzentration;

Fig. 7      Beispiele eines "visuellen Lungenindexes" als graphische Darstellung der Alveolenrekrutierungsrate g'(x), wobei die Alveolenrekrutierungsrate g'(x) als Dichteverteilung eines Kreises dargestellt wird, dessen Radius den Betrag von g'(x) darstellt,

Fig. 8A      eine beispielhafte gemessene Kohlendioxidkonzentration $f_{mess}$ in Atemluft als Funktion der Zeit über mehrere Atemzüge;

Fig. 8B      ein gemessener, "unkorrigierter" Gasfluß *Flow* als Funktion der Zeit, welcher um eine Verzögerung $\Delta x$ gegenüber der Gaskonzentration $f_{mess}$ verzögert ist; und

Fig.8C      der zeitlich mit der Kohlendioxidkonzentration $f_{mess}$ synchronisierte Gasfluß *Flow;*

Fig. 9      einen Graph, welcher beispielhaft die Werte der Verzögerung $\Delta t$ im Verlaufe eines Belastungsversuchs gegen die Zeit;

Fig. 10A      prozentuale Abweichungen zwischen experimentellen Expirogrammdaten $f_{mess}$ und herkömmlichen Fitwerten, und

Fig. 10B      prozentuale Abweichungen zwischen experimentellen Expirogrammdaten $f_{mess}$ und Fitwerten gemäß der vorliegenden Erfindung darstellt.

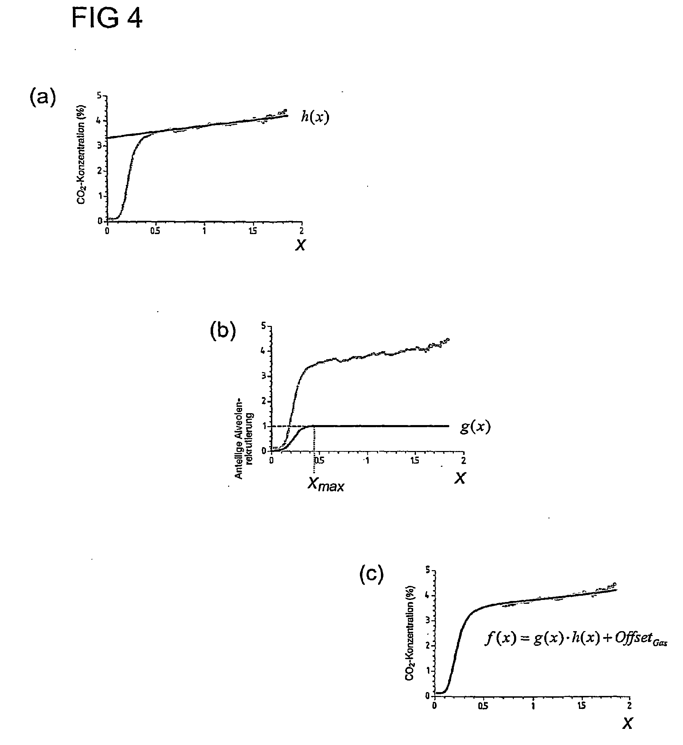

**Fig. 4** zeigt an einem beispielhaften Expirogramm für das Atemgas $CO_2$, welches experimentell erhalten wurde, die Durchführung einer bevorzugten Variante des erfindungsgemäßen Verfahrens. Die experimentell ermittelten Expirogrammdaten, d.h. die am Mund gemessenen $CO_2$-Konzentration gegen das ausgeatmete Atemvolumen werden durch eine nichtlineare Fitfunktion $f(x)$ gefittet, welche sich aus einem physiologisch begründeten Produkteinsatz und einem konstanten Offset zusammensetzt.

Wie in Fig. 4(a) dargestellt ist, beschreibt die lineare Funktion $h(x)$ nach erfolgtem Fitting näherungsweise die Expirogrammdaten in dem linear ansteigenden Kurventeil des Expirogramms, d.h. im Bereich der "Alveolar Slope". Der zweite Produktterm des Produktansatzes der Fitfunktion $f(x)$, d.h. die nichtlineare Funktion $g(x)$, stellt demgegenüber die anteilige Alveolenrekrutierung dar. Bei der Funktion $g(x)$ handelt es sich vorzugsweise um eine doppelsigmoidale Funktion, welche monoton steigend und stetig differenzierbar ist. Fig. 4(c) zeigt eine Überlagerung der gemessenen Expirogrammdaten und der Fitfunktion $f(x)$.

Insbesondere die numerisch robuste Berechnung des physiologischen Totraums $x_{VD}$ von Atemzug zu Atemzug bringt einen deutlichen Informationsgewinn in der Pneumologie, Intensivmedizin, Notfallmedizin, Physiologie, Sportmedizin, Arbeitsmedizin. Krankheitsbilder wie Asthma (anfallsweise Verengung der Atemwege), Atelekteasen (Akutverschluß von Atemwegen insbesondere bei beatmeten Patienten), Lungenembolie (Akutverschluß von Blutgefäßen der Lunge) oder auch eine uneffektive Atemmechanik bei sportlicher Belastung wirken sich auf den physiologischen Totraum $x_{VD}$ oder das Verhältnis $x_{VT}/x_{VD}$ aus.

Jedoch sind auch andere Formeigenschaften der Expirogramme von grundsätzlichem, insbesondere auch nicht-diagnostischem Interesse. Hierzu gehört beispielsweise die Steigerung der "Alveolaren Slopes" $b$ oder der tatsächliche Verlauf der alveolaren Gaskonzentration aus $h(x)$. Desweiteren ist auch die exponentielle Charakteristik der kurvigen Anteile der Expirogramme entweder gegen die Zeit oder gegen das Volumen für wissenschaftliche oder diagnostische Zwecke nutzbar.

Die Besonderheit der erfindungsgemäßen, multiplikativen Auftrennung der beiden Produktanteile der Fitfunktion $f(x)$ liegt zum einen in der numerischen Skalierung der physiologischen Basis und zum anderen in der besseren Möglichkeit eines nichtlinearen Fittings. Bisherige Anwendungen zur automatisierten Bewertung von "Alveolar Slopes" und des physiologischen Totraums $x_{VD}$ versuchten eine direkte Extraktion der linearen Anteile der Expirogramme. Dieser Ansatz ist jedoch in der Regel wegen der fehlenden Eindeutigkeit der Grenze zwischen dem linearen und dem kurvigen Abschnitt der Expirogramme wenig zufriedenstellend.

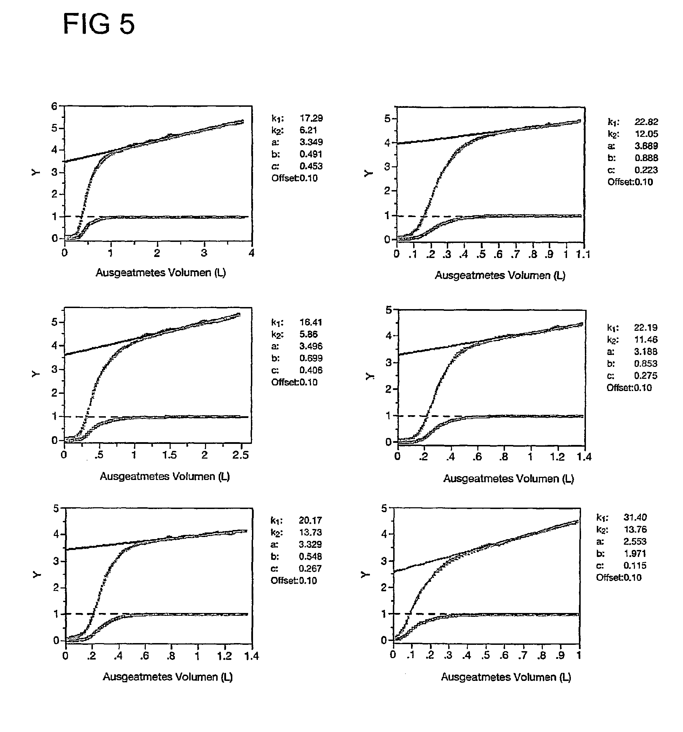

Im Gegensatz hierzu ermöglicht die Erfindung eine numerisch stabile, reproduzierbare und robuste Auswertung von Expirogrammen. Dies wird exemplarisch anhand von sechs zufälligen Einzelbeispielen von experimentell bestimmten Expirogrammen und den dazugehörigen Fits gemäß einem bevorzugten erfindungsgemäßen Verfahren in **Fig. 5** dargestellt. In Fig. 5(a) bis (f) ist jeweils das experimentelle Expirogramm gemeinsam mit dem linearen Produktterm $h(x)$ und dem nichtlinearen Produktterm $g(x)$ dargestellt. Die sich aus dem nichtlinearen Fitting ergebenden Parameter sind neben den jeweiligen Beispielsgraphen dargestellt.

Die Optimierung der Funktionsparameter beim Kurvenfitting auf den Verlauf der gemessenen Expirogrammdaten kann durch Anwendung verschiedener, etablierter Verfahren geschehen. Bevorzugt ist eine Parameteroptimierung mit Hilfe des Marquardt-Levenberg-Verfahrens. Die Robustheit der Parameteroptimierung nach dem Marquardt-Levenberg-Verfahren nimmt mit zunehmender Parameterzahl ab. Umgekehrt wird das Verfahren robuster, je exakter die zu schätzenden Startwerte in der Nähe des tatsächlichen Optimums liegen. Gemäß einem besonders bevorzugten erfindungsgemäßen Verfahren wird daher das erfindungsgemäße Verfahren derart implementiert, daß es stufenweise vom einfachsten Modell für die Ermittlung der "Alveolar Slopes" zu jeweils komplexeren Modellen mit zunehmender Parameterzahl übergeht und die dadurch erzielten Ergebnisparameter jeweils als Startwert in das jeweils nächst komplexere Modell überführt.

Die Gasmeßsonde einer erfindungsgemäßen Vorrichtung kann beispielsweise ein auf Basis respiratorischer Massenspektroskopie arbeitender Gassensor sein. Gleichwohl ist die technische Anwendung des erfindungsgemäßen Verfahrens oder einer erfindungsgemäßen Vorrichtung auch mit sonstigen Verfahren der fortlaufenden Gasanalyse und Gasvolumenmessung durchführbar. Insbesondere stehen für die Kohlendioxidkonzentrationsmessung in der Atemluft vielfältige und einfache Verfahren zur Verfügung. Eine Datenerfassungsrate von 25 Hertz oder mehr wird für eine saubere Meßdatenerfassung von Expirogrammen bevorzugt.

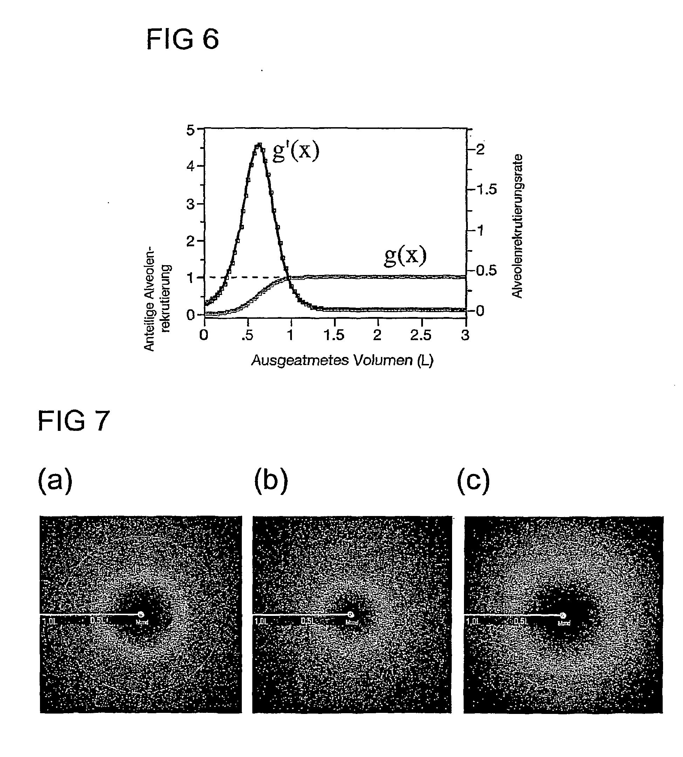

**Fig. 6** zeigt die anteilige Alveolenrekrutierung $g(x)$ als Funktion des ausgeatmeten volumens sowie die erste Ableitung von $g(x)$, welche die Alveolenrekrutierungsrate $g'(x)$ darstellt. $g(x)$ beschreibt den Anteil des Alveolarraums, der bei dem Volumen x begonnen hat, sich in der Ausatmung zu entleeren. Je mehr Alveolarraum auf diese Weise rekrutiert wurde, je mehr Totraum von Alveolarluft ausgewaschen wurde, desto näher liegt der Wert von $g(x)$ bei einem konstanten Wert, welcher vorzugsweise gleich 1 gesetzt wird. $g(x) = 1$ bedeutet, daß 100% des Totraums von Alveolarluft ausgewaschen wurde und daß in diesem Moment die tatsächliche Gaskonzentration der Alveolen ohne Verfälschung durch Einmischung von Totraumluft meßbar ist. Die Alveolenrekrutierungsrate $g'(x)$ bezeichnet die Rate des jeweils beim Volumen x zu Entleerung hinzukommenden Alveolarraums. Je höher der Wert für $g'(x)$ ist, desto mehr Gas aus dem Alveolarraum hat bei diesem Atemvolumen jeweils den dazugehörigen Totraum ausgewaschen.

Die graphische Darstellung und numerische Skalierung der Alveolenrekrutierungsrate $g'(x)$ bedeutet einen wesentlichen Informationsgewinn, da verschiedene pathologische oder physiologische Veränderungen der Lunge aus dieser Form der Darstellung diagnostizierbar sind. Veränderungen in der Atemmechanik oder der Lungenanatomie hätten einen direkten Einfluß auf die Alveolenrekrutierungsrate $g'(x)$.

Besonders bevorzugt wird aus der Alveolenrekrutierungsrate $g'(x)$ eine intuitiv erfaßbare Darstellung im Sinne eines "visuellen Lungenindex" erstellt. In einer solchen bevorzugten, graphischen Darstellung wird die Verteilung des Totraums, d.h. die "Entfernung" der jeweiligen Alveolen zum Mund, dargestellt.

Eine bevorzugte graphische Darstellung der Alveolenrekrutierungsrate $g'(x)$ stellt die symbolhafte Übertragung der Information von $g'(x)$ auf die Dichte von im Kreis angeordneten Punkten dar, wie es in **Fig. 7** dargestellt ist. Die Punktdichte in einem bestimmten Abstand zum Mittelpunkt der Grafik repräsentiert die Wahrscheinlichkeit der Lage von Alveolen/Austauschraum $x_{VD}$ ergibt somit eine visuelle Information über den physiologischen Totraum $x_{VD}$ und die Verteilung dieses Totraums innerhalb der Lunge mit jedem Atemzug. Die Streuung der zufallsverteilten Punkte repräsentiert somit die Verteilung der Alveolarraumrekrutierung.

Besonders vorteilhaft wäre eine fortlaufende bzw. zeitnahe Berechnung und graphische Darstellung der Alveolenrekrutierungsrate $g'(x)$ für Monitoringzwecke, beispielsweise für beatmete Patienten. Eine Verlegung von Luftwegen oder Verengung der Luftwege durch eine sogenannte Bronchokonstriktion wäre *prima vista* in der veränderten Form erkennbar. Weitere Anwendungsgebiete sind Untersuchungen zur Reaktion auf körperliche Belastung in der Sportmedizin oder Arbeitsmedizin, bronchiale Provokationstests in der Pneumologie, vergleichende Untersuchungen bei chronisch obstruktiver Lungenerkrankung (COPD) oder bei Emphysem. Die Besonderheit des beschriebenen Verfahrens liegt in der fehlenden Notwendigkeit von Atemmanövern. Hierdurch entfällt vorteilhafterweise der Unsicherheitsfaktor der Probandenmitarbeit und der Probandenmotivation.

Die graphische Darstellung, wie Sie beispielhaft in Fig. 7 gezeigt ist, kann vorzugsweise durch verschiedene Basisinformationen ergänzt werden, die insbesondere durch entsprechende Markierungen in der Graphik (Atemzug für Atemzug) sichtbar gemacht werden können. Vorzugsweise wäre die Einbeziehung zumindest eines der folgenden, zusätzlichen Basisinformation:

- physiologischer Totraum:

  - gleitender Mittelwert zum Trennvergleich
  - Maximalwert (zurücksetzbar)
  - Minimalwert (zurücksetzbar)
  - verschiedene Perzentielenstufen der Alveolarraumrekrutierung

    (beispielsweise 5,25,75,90)
- maximale Atemzugtiefe $x_{VT}$

Diese Werte können z.B. als Farbtransparenz in die obige Diagrammform eingeblendet werden. Die graphische Umsetzung der Alveolenrekrutierungsrate $g'(x)$ für einen visuellen Lungenindex kann jedoch auch mittels anderer graphischer, intuitiv erfaßbarer Darstellungsformen erfolgen. Neben einer besonders suggestiven Kreisform wäre auch eine Verteilung der rechnerischen Information auf ein Bild der Lunge oder auf eine beliebige andere zwei- oder dreidimensionale Form möglich. Auch eine Darstellung als Histogramm kann vorteilhaft sein. Neben der linearen Skalierung der abzubildenden Fläche wäre auch eine logarithmische Skala zur Darstellung sinnvoll.

Neben der zeitnahen "online"-Darstellung der Alveolenrekrutierungsrate $g'(x)$ kann auch eine "offline"-Dokumentation der Atemzüge von Vorteil sein. Diese enthält in einem Ausdruck oder einer Bilddatei die Aufreihung in Ergebnisgraphiken des "visuellen Lungenindex". Hierdurch werden Veränderungen im Zeitverlauf nachträglich auswertbar. Über die Aufreihung wird im Ausdruck oder in der Bilddatei Vergleichslinien, z.B. im Bereich des Wertes des durchschnittlichen physiologischen Totraums $x_{VD}$ gelegt. Hierdurch können Veränderungen auf einen Blick erfaßt werden.

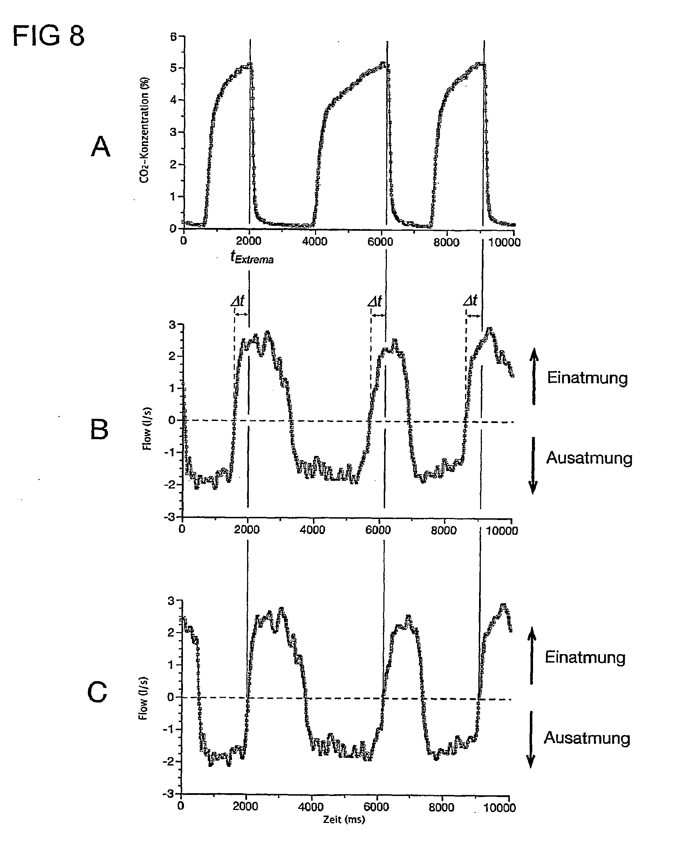

In **Figur 8** ist ein weiter Aspekt der Erfindung, weicher sich auf die Synchronisierung des gemessenen Gasflusses *Flow(t)* mit der Gaskonzentration $f_{mess}(t)$ bezieht, näher dargestellt. Fig. 8A zeigt die gemessene Kohlendioxidkonzentration in ausgeatmeter Luft für drei Atemzüge als Funktion der Zeit. Gleichsam könnte jedoch die Auftragung von Fig. 8 auch gegen das Atemvolumen erfolgen.

Der zugeordnete Gasfluß der Atemluft gemessen am Mund ist in Fig. 8B dargestellt.

Der Gasfluß ist insbesondere aufgrund unterschiedlicher Wegstrecken von Rohren bzw. Schläuchen für die Atemluft zu der Gasmeßsonde für die Gaskonzentrationsmessung und zu der Gasflußmeßsonde zur Messung des Gasflusses um einen Betrag $\Delta t$ verschoben. Erfindungsgemäß wird vorgeschlagen, insbesondere für jeden Atemzug den Gasfluß *Flow(t)* mit der Gaskonzentration $f_{mess}(t)$ zu synchronisieren, so daß die Nullstelle $t_B$ der aufsteigenden Flanke des Gasflusses *Flow(t)* mit dem zugeordneten Wert $t_{Extrema}$, bei welchem $f_{mess}(t)$ ein Maximum aufweist, zusammenfällt.

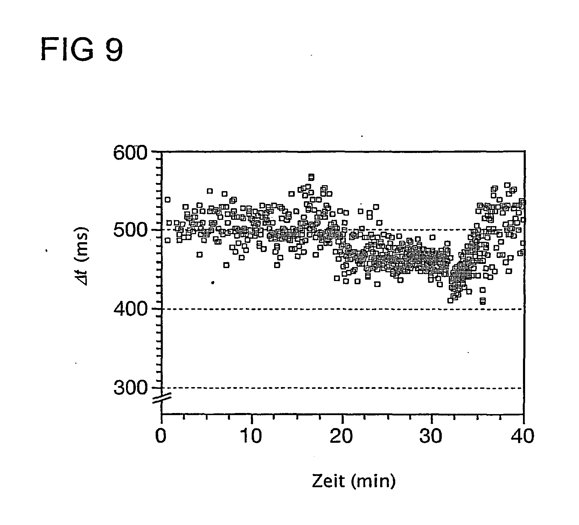

**Figur 9** zeigt einen Graph, welcher beispielhaft die Werte der Verzögerung $\Delta x$ im Verlaufe eines Belastungsversuchs gegen die Zeit darstellt. Erkennbar ist die deutlich Streuung der Verzögerung $\Delta t$ von Atemzug zu Atemzug, so daß eine atemzugsweise Bestimmung vorteilhaft ist.

Eine besonders vorteilhafte Anwendungsmöglichkeit der erfindungsgemäßen Vorrichtungen, Verfahren und Computerprogrammprodukte ist die Überwachung der Steuerung der Beatmung von Komatösen oder narkotisierten Patienten. Insbesondere ist durch die Erfindung eine bessere Einstellung einer schonenden, d.h. "protektiven" Beatmung möglich, da durch die automatisierbare Expirogrammanalyse Veränderungen der Lungenmechanik und -belüftung sofort sichtbar werden würden.

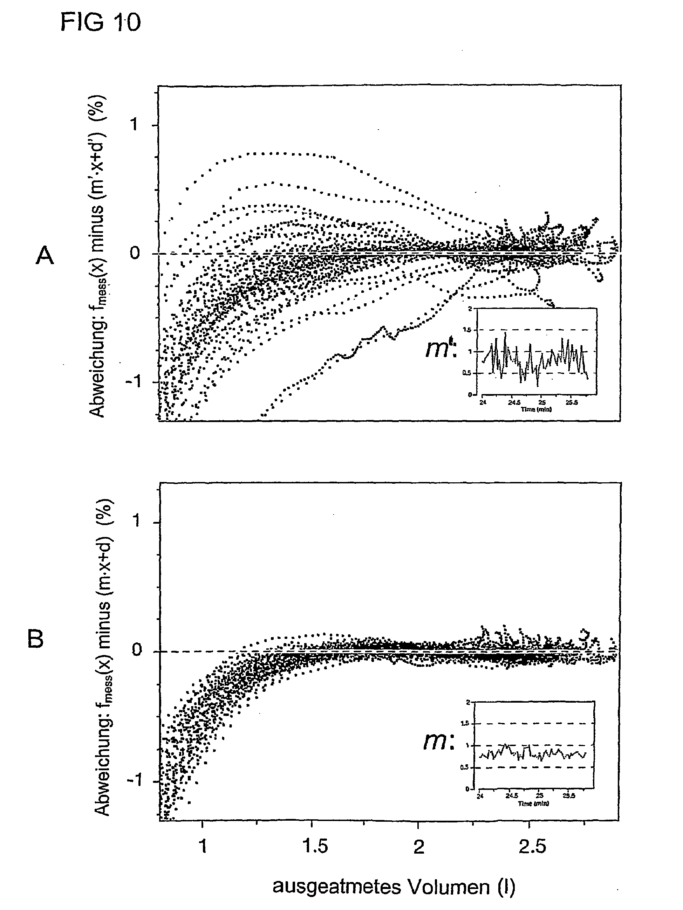

Die Verbesserungen in der Zuverlässigkeit, Reproduzierbarkeit und Robustheit der Auswertung von experimentell ermittelten Expirogrammen durch eine Vorrichtung bzw. ein Verfahren gemäß der vorliegenden Erfindung bzw. einer ihrer bevorzugten Ausführungsformen im Vergleich zu herkömmlichen Auswertungen ist in **Figur 10** dargestellt. Darin sind für die $CO_2$-Expirogramme von etwa 60 aufeinander folgenden Atemzügen die prozentualen Abweichungen zwischen den gemessen Werten $f_{mess}(x_1),...,f_{mess}(x_N)$ und entsprechenden gefitteten Werten dargestellt. Insbesondere zeigt Figur 10 A die prozentualen Abweichungen zwischen den gemessenen Werten $f_{mess}(x_1),...,f_{mess}(x_N)$ und herkömmlichen Fitwerten $m' \cdot x_1 + d',..., m' \cdot x_N + d'$ in Abhängigkeit vom ausgeatmeten Volumen, wobei für die herkömmlichen Fitwerte lediglich der linear ansteigende Kurventeil des Expirogramms mit einer Geraden $m' \cdot x + d'$ mit der Steigung $m'$ gefittet wurde. Im Vergleich dazu zeigt Figur 10 A die Abweichungen derselben experimentellen Expirogrammdaten $f_{mess}(x_1),...,f_{mess}(x_N)$ von den Funktionswerten $m \cdot x_1 + d,..., m \cdot x_1 + d$ einer Geraden $m \cdot x + d$ mit der Steigung $m$, welche dem linearen Anteil $h(x) + Offest_{co2}$ einer Fitfunktion $f(x) = g(x) \cdot h(x) + Offset_{Gas}$ mit $h(x) = m \cdot x + a$ gemäß einer bevorzugten Ausführungsform der vorliegenden Erfindung entspricht. Dabei wurde als stetig differenzierbare, nichtlineare Funktion $g(x)$ insbesondere eine Funktion gemäß Gleichung (5) mit $\lim_{x \to \infty} g(x) = 1$ verwendet.

Während die Daten der herkömmlichen Auswertung (Figur 10 A) von Atemzug zu Atemzug sehr stark streuen, sind sie für die erfindungsgemäße Auswertung (Figur 10 B) sehr viel besser reproduzierbar. Aus dem Vergleich des herkömmlichen Fits gemäß Figur 10 A und einem erfindungsgemäßen Fit (Figur 10 B) ist somit erkennbar, daß mit der vorliegenden Erfindung die Abweichungen der Fitdaten von den experimentellen Daten sowie die Abweichungen der Fitdaten verschiedener Atemzügen voneinander deutlich verringert und damit die Robustheit und Reproduzierbarkeit der Auswertung der Expirogramme deutlich verbessert werden kann. Dies ermöglicht eine zuverlässigere Bewertung des Zustands der untersuchten Lunge.

## Bezugszeichenliste

10   Außenluft
12   Atemweg
14   Alveolen
16   Kapillare
18   Lunge

## Patentansprüche

**1.** Vorrichtung zur Erfassung und Auswertung von Expirogrammen, umfassend:

- eine Gasmeßsonde, welche zur Ermittlung einer Gaskonzentration $f_{mess}$ eines Gases in ausgeatmeter Atemluft ausgelegt ist;
- eine Ausleseeinheit, welche mit der Gasmeßsonde in Signalverbindung steht und ausgelegt ist, für eine Vielzahl von Werten $x_1,...,x_N$ eines ausgeatmeten Volumens der ausgeatmeten Atemluft die zugeordneten ermittelten Gaskonzentrationen $f_{mess}(x_1),...,f_{mess}(x_N)$ aus der Gasmeßsonde auszulesen;
- eine Speichereinheit, welche zur Speicherung der den Werten $x_1,...,x_N$ zugeordneten Gaskonzentrationen $f_{mess}(x_1),...,f_{mess}(x_N)$ ausgelegt ist; **gekennzeichnet durch**
- eine Funktionsfiteinheit, welche mit der Speichereinheit in Signalverbindung steht und ausgelegt ist, eine nichtlineare Fitfunktion

$$f(x) = g(x) \cdot h(x) + Offset_{Gas}$$

für die gespeicherten Gaskonzentrationen $f_{mess}(x_1),...,f_{mess}(x_N)$ **durch** Bestimmen von Funktionen $g(x)$ und $h(x)$ zu bestimmen, wobei

    -- $h(x)=a+b \cdot x$ ist,

    -- $g(x)$ eine stetig differenzierbare, nichtlineare Funktion mit $g(0) = 0$ und $\lim\limits_{x \to \infty} g(x) = const$ ist,

    -- $a,b$ und $const$ Konstanten sind und

    -- $Offset_{Gas}$ eine konstante, mittlere Konzentration des Gases in Raumluft ist.

2. Vorrichtung nach Anspruch 1, weiter umfassend

    - eine Auswerteeinheit, welche zur Bestimmung des physiologischen Totraums $x_{VD}$ einer zugeordneten Lunge (18) ausgelegt ist, wobei

$$\int_0^{x_{VD}} g(x)h(x)dx = \lim_{x_{max} \to \infty} \int_{x_{VD}}^{x_{max}} h(x)(1 - g(x))dx$$

    gilt.

3. Vorrichtung nach einem der vorangegangenen Ansprüche, wobei die Funktion $g(x)$ eine monotone Funktion ist.

4. Vorrichtung nach Anspruch 3, wobei

$$g(x) = \cfrac{1}{1 + \cfrac{e^{(k_1 \cdot c - k_1 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} - \cfrac{e^{(k_2 \cdot c - k_2 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} + k_3$$

    gilt, wobei $k_1, k_2, k_3$ und $c$ Konstanten sind.

5. Vorrichtung nach einem der vorangegangenen Ansprüche, wobei die Funktionsfiteinheit ausgelegt ist, die Fitfunktion $f(x) = g(x) \cdot h(x) + Offset_{Gas}$ nach dem Marquardt-Levenberg-Algorithmus zu bestimmen.

6. Vorrichtung nach einem der vorangegangenen Ansprüche, wobei die Speichereinheit eine elektronische Speichereinheit ist.

7. Vorrichtung nach einem der vorangegangenen Ansprüche, ferner umfassend:

    - eine Auswerteeinrichtung, welche zur Berechnung der Alveolenrekrutierungsrate $g'(x)$ durch Ableiten der Funktion $g(x)$ nach dem ausgeatmeten Volumen $x$ ausgelegt ist.

8. Vorrichtung nach Anspruch 7, ferner umfassend:

    - eine Anzeigevorrichtung, welche ausgelegt ist, die Alveolenrekrutierungsrate $g'(x)$ graphisch darzustellen.

**9.** Verfahren zur Erfassung und Auswertung von Expirogrammen, umfassend folgende Schritte:

- Messen von Gaskonzentrationen $f_{mess}(x_1),...,f_{mess}(x_N)$ für eine Vielzahl von Werten $x_1,...,x_N$ eines ausgeatmeten Volumens ausgeatmeter Atemluft;
- Speichern der den Werten $x_1...x_N$ zugeordneten, gemessenen Gaskonzentrationen $f_{mess}(x_1),...,f_{mess}(x_N)$; **gekennzeichnet durch** das
- Bestimmen einer nichtlinearen Fitfunktion

$$f(x) = g(x) \cdot h(x) + Offset_{Gas}$$

für die gespeicherten Gaskonzentrationen $f_{mess}(x_1),...f_{mess}(x_N)$ **durch** Bestimmen von Funktionen $g(x)$ und $h(x)$, wobei

-- $h(x)=a+b\cdot x$ ist,

-- $g(x)$ eine stetig differenzierbare, nichtlineare Funktion mit $g(0) = 0$ und $\lim_{x \to \infty} g(x) = const$ ist,

-- $a,b$ und $const$ Konstanten sind und
-- $offset_{Gas}$ eine konstante, mittlere Konzentration des Gases in Raumluft ist.

**10.** Verfahren nach Anspruch 9, weiter umfassend den Schritt

- Bestimmen des physiologischen Totraums $x_{VD}$ einer zugeordneten Lunge (18), wobei

$$\int_0^{x_{VD}} g(x)h(x)dx = \lim_{x_{max} \to \infty} \int_{x_{VD}}^{x_{max}} h(x)(1 - g(x))dx$$

gilt.

**11.** Verfahren nach Anspruch 9 oder 10, wobei

$$g(x) = \cfrac{1}{1 + \cfrac{e^{(k_1 \cdot c - k_1 \cdot x)}}{1 + e^{\left(\frac{2k_1 \cdot c \cdot k_2}{|k_1 + k_2|} + \frac{2k_1 \cdot k_2 \cdot x}{|k_1 + k_2|}\right)}} - \cfrac{e^{(k_2 \cdot c - k_2 \cdot x)}}{1 + e^{\left(-\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} + k_3$$

gilt, wobei $k_1, k_2, k_3$ und $c$ Konstanten sind.

**12.** Verfahren nach einem der Ansprüche 9 bis 11, wobei der Schritt des Bestimmens der Fitfunktion $f(x) = g(x) \cdot h(x) + Offset_{Gas}$ einen Marquardt-Levenberg-Algorithmus umfaßt.

**13.** Verfahren nach einem der Ansprüche 9 bis 12, ferner umfassend den Schritt:

- Berechnen der Alveolenrekrutierungsrate $g'(x)$ durch Ableiten der Funktion $g(x)$.

**14.** Verfahren nach Anspruch 13, ferner umfassend den Schritt:

- Graphisches Darstellen der Alveolenrekrutierungsrate $g'(x)$ auf einer Anzeigevorrichtung.

15. Computerprogrammprodukt zur Auswertung von Expirogrammen, welches Programmteile zur Durchführung eines Verfahrens mit folgenden Schritten umfaßt:

- Eingeben von Gaskonzentrationen $f_{mess}(x_1), ..., f_{mess}..(x_N)$ für eine Vielzahl von Werten $x_1,...,x_N$ eines ausgeatmeten Volumens ausgeatmeter Atemluft;
- Speichern der den Werten $x_1,...,x_N$ zugeordneten, gemessenen Gaskonzentrationen $f_{mess}(x_1),..., f_{mess}(x_N)$ ;
**gekennzeichnet durch**
- Bestimmen einer nichtlinearen Fitfunktion

$$f(x) = g(x)\cdot h(x) + Offset_{Gas}$$

für die gespeicherten Gaskonzentrationen $f_{mess}(x_1),..., f_{mess}(x_N)$ **durch** Bestimmen von Funktionen g(x) und $h(x)$, wobei

  -- $h(x)=a+b\cdot x$ ist,

  -- $g(x)$ eine stetig differenzierbare, nichtlineare Funktion mit $g(0) = 0$ und $\lim_{x\to\infty} g(x) = const$ ist,

  -- $a,b$ und *const* Konstanten sind und
  -- $Offset_{Gas}$ eine konstante, mittlere Konzentration des Gases in Raumluft ist.

16. Computerprogrammprodukt nach Anspruch 15, weiter umfassend Programmteile zur Durchführung eines Verfahrens mit dem Schritt

- Bestimmen des physiologischen Totraums $x_{VD}$ einer zugeordneten Lunge, wobei

$$\int_0^{x_{VD}} g(x)h(x)dx = \lim_{x_{max}\to\infty} \int_{x_{VD}}^{x_{max}} h(x)(1 - g(x))dx$$

gilt.

## Claims

1. An apparatus for detecting and evaluating expirograms, comprising:

- a gas measuring probe designed to determine a gas concentration $f_{mess}$ of a gas in exhaled respiratory air;
- a read-out unit in signal connection with the gas measuring probe and designed to read out the associated detected gas concentrations $f_{mess}(x_1),...,f_{mess}(x_N)$ from the gas measuring probe for a multitude of values $x_1,..., x_N$ of an exhaled volume of the exhaled respiratory air;
- a storage unit designed to store the gas concentrations $f_{mess}(x_1),..., f_{mess}(x_N)$ associated with the values $x_1,..., x_N$ ;
**characterized by**
- a function fit unit in signal connection with the storage unit and designed to determine a non-linear fit function

$$\hat{f}(x) = g(x)\cdot h(x) + Offset_{Gas}$$

for the stored gas concentrations $f_{mess}(x_1),...,f_{mess}(x_N)$ by determining functions $g(x)$ and $h(x)$, wherein

-- $h(x)=a+b \cdot x,$

-- $g(x)$ is a continuously differentiable, non-linear function with $g(0) = 0$ and $\lim\limits_{x \to \infty} g(x) = const$,

-- $a,b$ and $const$ are constants, and

-- $Offset_{Gas}$ is a constant mean concentration of the gas in indoor air.

2. The Apparatus according to claim 1, further comprising

- an evaluating unit designed to determine the physiologic dead space $x_{VD}$ of an associated lung (18), wherein it holds that

$$\int_0^{x_{VD}} g(x)h(x)dx = \lim_{x_{max} \to \infty} \int_{x_{VD}}^{x_{max}} h(x)(1 - g(x))dx.$$

3. The Apparatus according to one of the preceding claims, wherein the function g(x) is a monotonic function.

4. The Apparatus according to claim 3, wherein it holds that

$$g(x) = \cfrac{1}{1 + \cfrac{e^{(k_1 \cdot c - k_1 \cdot x)}}{1 + e^{\left(\frac{2k_1 \cdot c \cdot k_2}{|k_1 + k_2|} + \frac{2k_1 \cdot k_2 \cdot x}{|k_1 + k_2|}\right)}} - \cfrac{e^{(k_2 \cdot c - k_2 \cdot x)}}{1 + e^{\left(\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} + k_3$$

wherein $k_1, k_2, k_3$ and c are constants.

5. The Apparatus according to one of the preceding claims, wherein the function fit unit is designed to determine the fit function $f(x) = g(x) \cdot h(x) + Offset_{Gas}$ according to the Levenberg-Marquardt algorithm.

6. The Apparatus according to one of the preceding claims, wherein the storage unit is an electronic storage unit.

7. The Apparatus according to one of the preceding claims, further comprising:

- an evaluating device designed to calculate the alveolar recruitment rate $g'(x)$ by deriving the function $g(x)$ according to the exhaled volume $x$.

8. The apparatus according to claim 7, further comprising:

- a display device designed to graphically illustrate the alveolar recruitment rate $g'(x)$.

9. A method for detecting and evaluating expirograms, comprising the following steps:

- measuring gas concentrations $f_{mess}(x_1),...,f_{mess}(x_N)$ for a multitude of values $x_1,...,x_N$ of an exhaled volume of exhaled respiratory air;
- storing the measured gas concentrations $f_{mess}(x_1),...,f_{mess}(x_N)$ associated with the values $x_1,...,x_N$;
**characterized by**
- determining a non-linear fit function

$$f(x) = g(x) \cdot h(x) + Offset_{Gas}$$

for the stored gas concentrations $f_{mess}(x_1),...,f_{mess}(x_N)$ by determining functions $g(x)$ and $h(x)$, wherein

-- $h(x)=a+b\cdot x,$

-- $g(x)$ is a continuously differentiable, non-linear function with $g(0) = 0$ and $\lim\limits_{x\to\infty} g(x) = const$,

-- $a,b$ and $const$ are constants, and

-- $Offset_{Gas}$ is a constant mean concentration of the gas in indoor air.

**10.** The method according to claim 9, further comprising the step of

- determining the physiologic dead space $x_{VD}$ of an associated lung (18), wherein it holds that

$$\int_0^{x_{VD}} g(x)h(x)dx = \lim_{x_{max}\to\infty} \int_{x_{VD}}^{x_{max}} h(x)\big(1 - g(x)\big)dx$$

**11.** The method according to claim 9 or 10, wherein it holds that

$$g(x) = \cfrac{1}{1 + \cfrac{e^{(k_1 \cdot c - k_1 \cdot x)}}{1 + e^{\left(\frac{2k_1 \cdot c \cdot k_2}{|k_1 + k_2|} + \frac{2k_1 \cdot k_2 \cdot x}{|k_1 + k_2|}\right)}} - \cfrac{e^{(k_2 \cdot c - k_2 \cdot x)}}{1 + e^{\left(\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} + k_3$$

wherein $k_1, k_2, k_3$ and $c$ are constants.

**12.** The method according to one of claims 9 to 11, wherein the step of determining the fit function $f(x)=g(x)\cdot h(x)+Offset_{Gas}$ comprises a Levenberg-Marquardt algorithm.

**13.** The method according to one of claims 9 to 12, further comprising the step of:

- calculating the alveolar recruitment rate $g'(x)$ by deriving the function $g(x)$.

**14.** The method according to claim 13, further comprising the step of:

- graphically illustrating the alveolar recruitment rate $g'(x)$ on a display device.

**15.** A computer program product for evaluating expirograms, comprising program parts for performing a method with the following steps:

- inputting gas concentrations $f_{mess}(x_1),...,f_{mess}(x_N)$ for a multitude of values $x_1,...,x_N$ of an exhaled volume of exhaled respiratory air;
- storing the measured gas concentrations $f_{mess}(x_1),...,f_{mess}(x_N)$ associated with the values $x_1,...,x_N$;
- determining a non-linear fit function

$$f(x) = g(x) \cdot h(x) + Offset_{Gas}$$

for the stored gas concentrations $f_{mess}(x_1),...,f_{mess}(x_N)$ by determining functions $g(x)$ and $h(x)$, wherein

-- $h(x)=a+b\cdot x,$

-- $g(x)$ is a continuously differentiable, non-linear function with $g(0) = 0$ and $\lim_{x\to\infty} g(x) = const$,

-- $a,b$ and $const$ are constants, and

-- $Offset_{Gas}$ is a constant mean concentration of the gas in indoor air.

16. The computer program product according to claim 15, further comprising program parts for performing a method with the step of

- determining the physiologic dead space $x_{VD}$ of an associated lung, wherein it holds that

$$\int_0^{x_{VD}} g(x)h(x)dx = \lim_{x_{max}\to\infty} \int_{x_{VD}}^{x_{max}} h(x)(1 - g(x))dx.$$

**Revendications**

1. Dispositif de détection et d'évaluation d'expirogrammes, comprenant :

- une sonde de mesure du gaz, qui est conçue pour la détermination d'une concentration de *gaz* $f_{mess}$ d'un gaz dans de l'air respirable expiré ;
- une unité de lecture, qui se trouve en liaison de signaux avec la sonde de mesure du gaz et est conçue pour lire, pour une pluralité de valeurs $x_1,...,x_N$ d'un volume expiré de l'air respirable expiré, les concentrations de gaz déterminées correspondantes $f_{mess}(x_1),...,f_{mess}(x_N)$ de la sonde de mesure du gaz ;
- une unité de mémoire, qui est conçue pour la mémorisation des concentrations de gaz $f_{mess}(x_1),... ,f_{mess}(x_N)$ correspondant aux valeurs $x_1,...,x_N$; **caractérisé par**
- une unité de bonne condition physique fonctionnelle, qui se trouve en liaison de signaux avec l'unité de mémoire et est conçue pour déterminer une fonction de bonne condition physique non linéaire

$$f(x) = g(x) \cdot h(x) + Offset_{Gas}$$

pour les concentrations de gaz mémorisées $f_{mess}(x_1),...,f_{mess}(x_N)$ en déterminant des fonctions $g(x)$ et $h(x)$, où
- $h(x) = a+b\cdot x,$

- $g(x)$ est une fonction dérivable continue non linéaire avec $g(0) = 0$ et $\lim_{x\to\infty} g(x) = const$,

- $a, b$ et $const$ sont des constantes et
- $Offset_{Gas}$ est une concentration moyenne constante du gaz dans l'air ambiant.

2. Dispositif selon la revendication 1, comprenant en outre

- une unité d'évaluation qui est conçue pour la détermination de l'espace mort $x_{VD}$ d'un poumon correspondant (18), dans lequel

$$\int_0^{x_{VD}} g(x)h(x)dx = \lim_{x_{max} \to \infty} \int_{x_{VD}}^{x_{max}} h(x)(1 - g(x))dx$$

est valable.

**3.** Dispositif selon l'une des revendications précédentes, dans lequel la fonction *g(x)* est une fonction monotone.

**4.** Dispositif selon la revendication 3, dans lequel

$$g(x) = \cfrac{1}{1 + \cfrac{e^{(k_1 \cdot c - k_1 \cdot x)}}{1 + e^{\left(\frac{2k_1 \cdot c \cdot k_2}{|k_1 + k_2|} + \frac{2k_1 \cdot k_2 \cdot x}{|k_1 + k_2|}\right)}} - \cfrac{e^{(k_2 \cdot c - k_2 \cdot x)}}{1 + e^{\left(\frac{2k_1 \cdot c \cdot k_2}{|k_1 + k_2|} + \frac{2k_1 \cdot k_2 \cdot x}{|k_1 + k_2|}\right)}} + e^{(c \cdot k_2 - k_2 x)}} + k_3$$

est valable, où $k_1$, $k_2$, $k_3$ et c sont des constantes.

**5.** Dispositif selon l'une des revendications précédentes, dans lequel l'unité de bonne condition physique fonctionnelle est conçue pour déterminer la fonction de bonne condition physique $f(x) = g(x) \cdot h(x) + Offset_{Gas}$ selon l'algorithme de Levenberg-Marquardt.

**6.** Dispositif selon l'une des revendications précédentes, dans lequel l'unité de mémoire est une unité de mémoire électronique.

**7.** Dispositif selon l'une des revendications précédentes, comprenant en outre :

- un dispositif d'évaluation qui est conçu pour le calcul du taux de recrutement alvéolaire *g'(x)* en dérivant la fonction *g(x)* selon le volume expiré x.

**8.** Dispositif selon la revendication 7, comprenant en outre :

- un dispositif d'affichage qui est conçu pour représenter graphiquement le taux de recrutement alvéolaire *g'(x)*.

**9.** Procédé de détection et d'évaluation d'expirogrammes, comprenant les étapes suivantes consistant à :

- mesurer des concentrations de gaz $f_{mess}(x_1),...,f_{mess}(x_N)$ pour une pluralité de valeurs $x_1,...,x_N$ d'un volume expiré d'air respirable expiré ;
- mémoriser les concentrations de gaz $f_{mess}(x_1),...,f_{mess}(x_N)$ mesurées correspondant aux valeurs $x_1,...x_N$ ;
**caractérisé par**
- la détermination d'une fonction de bonne condition physique non linéaire $f(x) = g(x) \cdot h(x) + Offset_{Gas}$ pour les concentrations de gaz mémorisées $f_{mess}(x_1),...,f_{mess}(x_N)$ en déterminant les fonctions *g(x)* et *h(x)*, où
- *h(x) = a+b·x,*

- *g(x)* est une fonction dérivable continue non linéaire avec *g(0) = 0* et $\lim_{x \to \infty} g(x) = const$,

- *a, b* et *const* sont des constantes et
- $Offset_{Gas}$ est une concentration moyenne constante du gaz dans l'air ambiant.

**10.** Procédé selon la revendication 9, comprenant en outre l'étape consistant à

- déterminer l'espace mort physiologique $x_{VD}$ d'un poumon correspondant (18), dans lequel

$$\int_0^{x_{VD}} g(x)h(x)dx = \lim_{x_{max}\to\infty} \int_{x_{VD}}^{x_{max}} h(x)\big(1 - g(x)\big)dx$$

est valable.

11. Procédé selon la revendication 9 ou 10, dans lequel

$$g(x) = \cfrac{1}{1 + \cfrac{e^{(k_1\cdot c - k_1\cdot x)}}{1 + e^{\left(\frac{2k_1\cdot c\cdot k_2}{|k_1 + k_2|} + \frac{2k_1\cdot k_2\cdot x}{|k_1 + k_2|}\right)}} - \cfrac{e^{(k_2\cdot c - k_2\cdot x)}}{1 + e^{\left(\frac{2k_1\cdot c\cdot k_2}{|k_1 + k_2|} + \frac{2k_1\cdot k_2\cdot x}{|k_1 + k_2|}\right)}} + e^{(c\cdot k_2 - k_2 x)}} + k_3$$

est valable, où $k_1$, $k_2$, $k_3$ et $c$ sont des constantes.

12. Procédé selon l'une des revendications 9 à 11, dans lequel l'étape consistant à déterminer la fonction de bonne condition physique $f(x) = g(x).h(x) + Offset_{Gas}$ comprend un algorithme de Levenberg-Marquardt.

13. Procédé selon l'une des revendications 9 à 12, comprenant en outre l'étape consistant à :

    - calculer le taux de recrutement alvéolaire $g'(x)$ en dérivant la fonction $g(x)$.

14. Procédé selon la revendication 13, comprenant en outre l'étape consistant à :

    - représenter graphiquement le taux de recrutement alvéolaire $g'(x)$ sur un dispositif d'affichage.

15. Produit programme informatique pour évaluer des expirogrammes, qui présente des parties de programme pour effectuer un procédé comprenant les étapes suivantes consistant à :

    - saisir des concentrations de gaz $f_{mess}(x_1),...,f_{mess}(x_N)$ pour une pluralité de valeurs $x_1,...,x_N$ d'un volume expiré d'air respirable expiré ;
    - mémoriser les concentrations de gaz $f_{mess}(x_1),...,f_{mess}(x_N)$ mesurées correspondant aux valeurs $x_1,...,x_N$ ;
    **caractérisé par**
    - déterminer une fonction de bonne condition physique non linéaire $f(x) = g(x)\cdot h(x) + Offset_{Gas}$
    pour les concentrations de gaz mémorisées $f_{mess}(x_1),...,f_{mess}(x_N)$ en déterminant les fonctions $g(x)$ et $h(x)$, où
    - $h(x) = a + b\cdot x,$

    - g($x$) est une fonction dérivable continue non linéaire avec $g(0) = 0$ et $\lim_{x\to\infty} g(x) = const$,

    - $a$, $b$ et $const$ sont des constantes et
    - $Offset_{Gas}$ est une concentration moyenne constante du gaz dans l'air ambiant.

16. Produit programme informatique selon la revendication 15, comprenant en outre des parties de programme pour effectuer un procédé avec l'étape consistant à

    - déterminer l'espace mort physiologique $x_{VD}$ d'un poumon correspondant, où

$$\int_0^{x_{VD}} g(x)h(x)dx = \lim_{x_{max}\to\infty} \int_{x_{VD}}^{x_{max}} h(x)\big(1 - g(x)\big)dx$$

est valable.

# FIG 1

10

12

14

16

# FIG 2

FIG 3

# FIG 4

(a)

$h(x)$

(b)

$g(x)$

$X_{max}$

(c)

$$f(x) = g(x) \cdot h(x) + Offset_{Gas}$$

# FIG 5

FIG 6

FIG 7

(a)                    (b)                    (c)

# FIG 8

FIG 9

## FIG 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **Fowler et al.** The Respiratory Dead Space. *Am J Physiol,* 1948, vol. 154, 405-416

- **A.H. Kars.** Dead space and slope indices from the expiratory carbon dioxide tension-volume curve. *Eur Respir J,* 1997, vol. 10, 1829-1836